# EUROPÄISCHE PATENTANMELDUNG

(11) **EP 3 811 992 A1**
(43) Veröffentlichungstag der Anmeldung: **28.04.2021**
(21) Anmeldenummer: 20208610.4
(22) Anmeldetag: 30.11.2005
(51) Int. Cl.: A61M 5/315

(54) **INJEKTIONSGERÄT MIT SPIRALFEDER**

(30) Priorität: 31.12.2004 DE 102004063645
(62) Teilanmeldung aus: 19187017.9
(71) Anmelder: Ypsomed AG, 3401 Burgdorf (CH)
(72) Erfinder: Meier, Christof, 3427 Utzenstorf (CH); Wittwer, Martin, verstorben (CH); Stettler, Peter, 3423 Ersigen (CH); Wittmann, Jürgen, 3400 Burgdorf (CH); Kuenzli, Daniel, 3074 Muri (CH); Kohlbrenner, Philippe, 3413 Kaltacker (CH)

(57) **Zusammenfassung**

Die Erfindung betrifft ein Injektionsgerät zur Ausschüttung eines fluiden Produkts durch eine Injektionsnadel, umfassend ein Gehäuse mit einem Reservoir (2) für das Produkt, eine zur Ausübung einer Ausschüttbewegung bewegbar gehaltene Kolbenstange (15), ein betätigbares oder auslösbares Antriebsglied (25) für die Kolbenstange (15), und eine Kupplung (6'-11') mit einem Kupplungseingangsglied (6; 6'), das in einem Kupplungseingriff das Antriebsglied (25) mit der Kolbenstange (15) koppelt und eine Antriebskraft des Antriebsglieds (5) auf die Kolbenstange (15) überträgt und die Ausschüttbewegung bewirkt. Das Antriebselement (25) ist eine als Drehfeder wirkende Spiralfeder.

## Beschreibung

Die Erfindung betrifft eine Vorrichtung zur Verabreichung eines fluiden Produkts. Die Vorrichtung kann insbesondere ein Injektionsgerät sein, vorzugsweise ein Injektionspen, bevorzugt für die Selbstverarbeitung. Injektionsgeräte sind beispielsweise aus der Diabetestherapie, der Verabreichung von Wachstumshormonen oder Osteoporosepräparaten bekannt. Derartige Geräte sollen einerseits die Gewähr dafür bieten, dass die richtige Dosis verabreicht wird, andererseits sollen die Geräte einfach und bequem bedienbar sein.

Ein aus der WO 97/10865 bekanntes Injektionsgerät weist zwei miteinander verschraubte Gehäuseteile auf. In einem der Gehäuseteile ist ein Reservoir mit einem zu verabreichenden Produkt aufgenommen, in dem anderen eine Förder- und Dosiereinrichtung. Die Förder- und Dosiereinrichtung umfasst einen in dem Reservoir aufgenommenen Kolben und eine Kolbenstange, die für die Ausschüttung einer einstellbaren Dosis in eine Vortriebsrichtung auf den Kolben wirkt. Die Förder- und Dosiereinrichtung umfasst ferner ein Antriebsglied, das von einem Benutzer für die Einstellung der Dosis und deren Ausschüttung betätigbar ist. Das Antriebsglied ist über zwei Spindeltriebe mit der Kolbenstange gekoppelt. Die Kolbenstange wird von dem das Reservoir aufnehmenden Gehäuseteil linear geführt. Nach Entleerung des Reservoirs muss der Benutzer für die erneute Verwendung mit einem gefüllten Reservoir die Kolbenstange zurückdrehen und dabei gleichzeitig auf das Antriebsglied drücken.

Ein Injektionsgerät mit einer der Kopplung von Kolbenstange und Antriebsglied vergleichbaren Mechanik ist aus der WO 99/38554 bekannt.

Es ist eine Aufgabe der Erfindung, eine Vorrichtung zur Verabreichung eines fluiden Produkts zu schaffen, die den Austausch eines entleerten Reservoirs auf einfache, aber dennoch sichere Weise gestattet.

Die Erfindung betrifft eine Vorrichtung zur Verabreichung eines fluiden Produkts, die wenigstens zwei lösbar miteinander verbundene Gehäuseteile aufweist, von denen ein erstes ein Reservoir für das Produkt oder vorzugsweise für ein Produktbehältnis und ein zweites einen Träger für eine Fördereinrichtung bildet. Die Fördereinrichtung ist vorzugsweise zu einer Förder- und Dosiereinrichtung weitergebildet, mit der eine auszuschüttende Dosis des Produkts einstellbar ist. Die Fördereinrichtung umfasst eine von dem zweiten Gehäuseteil gehaltene Kolbenstange und ein ebenfalls von dem zweiten Gehäuseteil gehaltenes Antriebsglied für die Kolbenstange. Das Antriebsglied ist mit der Kolbenstange so gekoppelt, vorzugsweise rein mechanisch, dass eine Betätigung oder Auslösung des Antriebsglieds automatisch eine Ausschüttbewegung der Kolbenstange bewirkt, durch die das Produkt ausgeschüttet wird. Das Produkt kann beispielsweise Insulin, ein Wachstumshormon, ein Osteoporosepräparat oder ein anderes Medikament oder grundsätzlich auch ein Nichtmedikament sein, das dem Menschen oder gegebenenfalls einem Tier verabreichbar ist. Die Fördereinrichtung umfasst ferner eine Kupplung, die in einem Kupplungseingriff das Antriebsglied mit der Kolbenstange koppelt.

Um das Reservoir beispielsweise im Falle der Entleerung möglichst einfach und rasch austauschen zu können, ist nach der Erfindung ein Entkopplungsglied vorgesehen, für das ebenfalls das zweite Gehäuseteil den Träger bildet und das im verbundenen Zustand der Gehäuseteile mit dem ersten Gehäuseteil gekoppelt ist, vorzugsweise durch einen mechanischen Eingriff, der unmittelbar zwischen dem Entkopplungsglied und dem ersten Gehäuseteil besteht. Die Kopplung ist so gebildet, dass das Entkopplungsglied durch eine Relativbewegung der Gehäuseteile zueinander, durch die die Gehäuseteile voneinander gelöst werden, relativ zu dem zweiten Gehäuseteil in eine Entkopplungsposition bewegt wird, in der es die Kolbenstange von dem Antriebsglied entkoppelt. Das Entkopplungsglied hält die Kupplung in einem ausgerückten Zustand, d.h. für die Ausschüttung im Kupplungseingriff befindliche Kupplungsglieder werden von dem Entkopplungsglied voneinander separiert und der Kupplungseingriff verhindert, falls nötig gelöst.

Wegen der Entkopplung kann bei einem Reservoirwechsel die üblicherweise ausgefahrene Kolbenstange zurückgesetzt, d.h. eingefahren werden, ohne auf das Antriebsglied zu wirken. Falls das Antriebsglied, wie dies bevorzugten Ausführungen entspricht, mit einer Dosisanzeige und/oder einem Dosierglied gekoppelt ist, wirkt die einfahrende Kolbenstange vorzugsweise auch nicht auf die Dosisanzeige und/oder das Dosierglied zurück. Der Benutzer muss dementsprechend nach einem Reservoirwechsel weder bei dem Antriebsglied noch bei einer oder einem gegebenenfalls vorhandenen Dosieranzeige oder Dosierglied Korrekturen vornehmen.

In bevorzugten Ausführungen, in denen die Vorrichtung mit der Fähigkeit ausgestattet ist, die mittels eines Ausschüttvorgangs ausschüttbare Dosis einzustellen, kann die Einstellbarkeit so gestaltet sein, dass sie einmalig beispielsweise von einem Arzt vorgenommen wird und der Benutzer sich dann pro Ausschüttvorgang stets die einmal eingestellte Dosis verabreicht. Bevorzugter ist die Dosis für jeden Ausschüttvorgang einzeln einstellbar, so dass ein Verwender, der sich das Produkt selbst verabreicht, die Dosen flexibel dem Bedarf angepasst einstellen kann. Eine Vorrichtung, die das Einstellen der Dosis gestattet, umfasst ein Dosierglied, das von dem Antriebsglied gebildet werden kann oder bevorzugter zusätzlich zu dem Antriebsglied vorgesehen und mit dem Antriebsglied so gekoppelt ist, vorzugsweise rein mechanisch, dass eine Dosierbewegung des Dosierglieds eine Dosierbewegung des Antriebsglieds zur Folge hat. In bevorzugter Ausführung ist eine Dosisanzeige zum Anzeigen der eingestellten Produktdosis vorgesehen. Die Anzeige kann eine akustische und/oder taktile und/oder insbesondere eine optische sein. Die Dosisanzeige ist mit dem Dosierglied und vorzugsweise auch mit dem Antriebsglied, falls das Antriebsglied nicht bereits das Dosierglied bildet, so gekoppelt, dass eine Bewegung, die das Dosierglied bei einer Einstellung der Produktdosis ausführt, eine Änderung der angezeigten Produktdosis bewirkt. Vorteilhafterweise entkoppelt das Entkopplungsglied in der Entkopplungsposition die Dosisanzeige und/oder das Dosierglied falls ein solches zusätzlich zum Antriebsglied vorgesehen ist, von der Kolbenstange, besonders bevorzugt auf die gleiche Weise wie es das Antriebsglied von dem Förderglied entkoppelt. Gegebenenfalls kann die Entkopplung jedoch auch an einer anderen Stelle einer zwischen dem Förderglied und dem Dosierglied und/oder dem Förderglied und der Anzeige bestehenden Kopplung herbeigeführt werden, indem die in diesem Fall weitere Kupplung an der betreffenden Schnittstelle in einem ausgerückten Zustand gehalten wird.

Für die Einstellung der Dosis ist das Antriebsglied vorzugsweise auch im verbundenen Zustand der Gehäuseteile von dem Förderglied entkoppelt. Falls die Vorrichtung zusätzlich mit einer Dosisanzeige und/oder einem Dosierglied ausgestattet ist, ist vorzugsweise auch die Dosisanzeige und/oder das Dosierglied für die Einstellung der Dosis von dem Förderglied entkoppelt, wenn die Gehäuseteile miteinander verbunden sind. In ebenfalls bevorzugter Ausführung kann eine Dosisanzeige und/oder ein Dosierglied für die Einstellung der Dosis von dem Förderglied entkoppelt sein, während das Antriebsglied während der Einstellung mit dem Förderglied gekoppelt ist. Die Entkopplung ermöglicht die freie Dosiswahl, d.h. die Dosis kann erhöht oder verringert werden ohne Rückwirkung auf die Kolbenstange.

Falls das Antriebsglied für die Einstellung der Dosis von dem Förderglied entkoppelt ist, wird es durch eine Kupplungsbewegung mit dem Förderglied gekoppelt, um dessen Ausschüttbewegung zu bewirken.

Die Antriebskraft wird in einer ersten Ausführung manuell und in einer zweiten Ausführung mittels eines Federantriebs, der in solch einer Ausführung das Antriebsglied bildet, aufgebracht. Obgleich weniger bevorzugt, kann die Antriebskraft auch motorisch aufgebracht werden.

Falls die Antriebskraft manuell aufgebracht wird, ist eine Wegstrecke der Antriebsbewegung in einen Kupplungswegabschnitt und einen sich anschließenden Ausschüttwegabschnitt unterteilt. Nach Zurücklegung des Kupplungswegabschnitts ist das Antriebsglied mit dem Förderglied gekoppelt und treibt danach über den Ausschüttwegabschnitt das Förderglied an. In der zweiten Ausführung, in der die Antriebskraft eine Federkraft oder gegebenenfalls eine Motorkraft ist, wird die Kupplungsbewegung vorzugsweise durch Betätigung eines Auslöseelements bewirkt. Sobald die Kupplung von einer für die Ausschüttung auf das Antriebsglied oder ein Auslöseelement auszuübenden Betätigungskraft oder einer motorischen oder einer gespeicherten Kraft, beispielsweise Federkraft, entlastet wird, löst sich die Kopplung vorzugsweise automatisch. Das Antriebsglied oder bevorzugter ein die Übertragung der Antriebsbewegung bewirkendes Kupplungsglied wird hierfür vorteilhafterweise mit einer der Kupplungsbewegung entgegenwirkenden Rückstellkraft beaufschlagt, um bei Entlastung des Antriebsglieds automatisch die Kopplung zwischen dem Antriebsglied und der Kolbenstange zu lösen. Denkbar wäre es alternativ auch, dass der Verwender die für den Antrieb der Kolbenstange geschlossene Kupplung durch eine manuell bewirkte Bewegung des Antriebsglieds oder alternativ eines weiteren Entkopplungsglieds öffnet, d.h. den Kupplungseingriff löst.

In einer Weiterentwicklung wird in einem Ruhezustand der Vorrichtung der mit der Kolbenstange verbundene, ausgangsseitige Teil der Kupplung an dem zweiten Gehäuseteil so festgelegt, dass die Kolbenstange keine Ausschüttbewegung ausführen kann, sondern bewusst freigegeben werden muss, beispielsweise unmittelbar im Zusammenhang mit einer Ausschüttung. Vorteilhafterweise wird die Kolbenstange mittels des Entkopplungsglieds festgelegt. Vorteilhaft ist es, wenn die Festlegung an dem Gehäuseteil durch die Kupplungsbewegung gelöst wird. Vorzugsweise wird in einer ersten Phase des Kupplungswegabschnitts der Antriebsbewegung des Antriebsglieds oder der Kupplungsbewegung eines Kupplungsglieds der Kupplungseingriff hergestellt und in einer sich anschließenden zweiten Phase die Festlegung an dem Gehäuse gelöst, vorteilhafterweise gegen die bereits genannte elastische Rückstellkraft. In bevorzugter Ausführung umfasst die Kupplung ein Kupplungszwischenglied, das einerseits mit dem zweiten Gehäuseteil, dem Entkopplungsglied oder einem anderen Element in einem die Festlegung an dem Gehäuseteil bewirkenden Eingriff ist und andererseits unmittelbar selbst oder über ein oder mehrere weitere Zwischenglieder mit der Kolbenstange in einem weiteren Eingriff ist, wobei im Ruhezustand der Vorrichtung das Kupplungszwischenglied sich in beiden Eingriffen befindet und nach Herstellung des Kupplungseingriffs nur noch in dem Eingriff mit oder zu der Kolbenstange.

Das Entkopplungsglied ist an dem zweiten Gehäuseteil bewegbar gehalten und mit dem ersten Gehäuseteil oder einer Adapterstruktur vorzugsweise in einem Führungseingriff. Für den Führungseingriff bildet entweder das Entkopplungsglied oder das erste Gehäuseteil eine Führungskurve und das andere ein von der Führungskurve geführtes Eingriffselement. Falls eine Adapterstruktur vorgesehen ist, gilt entsprechendes für den Führungseingriff, in dem dann das Entkopplungsglied vorzugsweise mit der Adapterstruktur ist. Bei dem Lösen der beiden Gehäuseteile wird das Entkopplungsglied durch den Führungseingriff relativ zu dem zweiten Gehäuseteil aus einer Ruheposition in die Entkopplungsposition und bei einem Verbinden der Gehäuseteile automatisch wieder in die Ruheposition bewegt. Die Führungskurve ist daher so geformt, dass die bei dem Lösen der Gehäuseteile stattfindende Relativbewegung der Gehäuseteile in dem formschlüssigen Führungseingriff in die Bewegung des Entkopplungsglieds relativ zu dem zweiten Gehäuseteil umgewandelt wird. Obgleich andere Mechanismen ebenfalls realisierbar wären, um das Entkopplungsglied bei dem Lösen der Gehäuseteile automatisch in die Entkopplungsposition zu überführen, beispielsweise mittels einer Federkraft, bietet die Ausbildung eines Führungseingriffs die Möglichkeit, den von dem Entkopplungsglied bis in die Entkopplungsposition zurückgelegten Weg und damit die Entkopplungsposition relativ zu dem zweiten Gehäuseteil vergleichsweise frei zu gestalten. Das Entkopplungsglied kann entgegen der Richtung, in die das erste Gehäuseteil von dem zweiten Gehäuseteil gelöst wird, in die Entkopplungsposition bewegt werden. Durch einen Führungseingriff kann das Entkopplungsglied vorteilhafterweise in der Entkopplungsposition sicher verrastet und die Rastverbindung durch das Verbinden der Gehäuseteile sicher gelöst werden. Insbesondere kann das Entkopplungsglied durch das Lösen der Gehäuseteile gegen die Richtung der Kupplungsbewegung, die den Kupplungseingriff herstellt, in die Entkopplungsposition bewegt werden.

Obgleich die Kupplung grundsätzlich unmittelbar zwischen dem Antriebsglied und der Kolbenstange gebildet sein kann, indem an den beiden Gliedern entsprechende Eingriffselemente geformt sind, die im Kupplungseingriff formschlüssig oder nur reibschlüssig oder form- und reibschlüssig miteinander verbunden sind, umfasst die Kupplung in bevorzugten Ausführungen ein Kupplungsausgangsglied das mit der Kolbenstange gekoppelt ist. Das Kupplungsausgangsglied wird bei der Produktausschüttung von dem Antriebsglied angetrieben und treibt auf die Kolbenstange ab. Das Entkopplungsglied entkoppelt in der Entkopplungsposition das Kupplungsausgangsglied von dem Antriebsglied, während die Kopplung mit der Kolbenstange bestehen bleibt. Das Kupplungsausgangsglied steht vorzugsweise unmittelbar mit der Kolbenstange in einem den Antrieb der Kolbenstange bewirkenden Eingriff. Der Eingriff kann rein formschlüssig oder rein kraftschlüssig sein. Vorzugsweise ist er form- und kraftschlüssig gebildet. Er kann insbesondere ein Gewindeeingriff sein, wobei die Gewindesteigung im Gewindeeingriff so groß ist, dass eine erzwungene Translationsbewegung der Kolbenstange in Richtung der Gewindeachse bei axial fixiertem Kupplungsausgangsglied möglich ist, d.h. der Gewindeeingriff ist nicht selbst hemmend.

Die Kupplung umfasst ein Kupplungseingangsglied, das mit dem Antriebsglied gekoppelt ist, vorzugsweise unmittelbar mit dem Antriebsglied in einem Eingriff steht. Die Kopplung kann rein formschlüssig oder rein kraftschlüssig sein. Vorzugsweise ist sie form- und kraftschlüssig und besonders bevorzugt als Gewindeeingriff gebildet. Der Gewindeeingriff ist vorzugsweise nicht selbst hemmend, so dass durch eine in Richtung der Gewindeachse auf das Antriebsglied wirkende Antriebskraft das Antriebsglied im Gewindeeingriff axial bewegbar ist.

In Ausführungen, in denen das Kupplungseingangsglied in einem ersten Gewindeeingriff angetrieben wird, vorzugsweise rotatorisch, und das Kupplungsausgangsglied über einen weiteren, zweiten Gewindeeingriff oder vorzugsweise in einem zweiten Gewindeeingriff unmittelbar mit einem Förderglied dieses antreibt, vorzugsweise translatorisch, bilden die beiden Gewindeeingriffe vorteilhafterweise ein Untersetzungsgetriebe, das die Wegstrecke der Antriebsbewegung in eine kürzere Wegstrecke der Ausschüttbewegung untersetzt. Die Untersetzung beträgt vorzugsweise wenigstens 2:1, noch bevorzugter wenigstens 3:1.

In bevorzugter Ausführung bleibt die Kopplung von Dosisanzeige und Antriebsglied im Kupplungseingriff bestehen, so dass eine der Dosierbewegung entgegengerichtete Antriebsbewegung des Antriebsglieds mit fortschreitender Ausschüttung in gleicher Weise fortschreitend zurückgestellt wird. Bei einem vorzeitigen Abbruch der Verabreichung, ob bewusst oder fehlerhaft unbewusst, zeigt die Dosisanzeige somit den noch nicht ausgeschütteten Rest der eingestellten Dosis an. Dies kann beispielsweise dann von Vorteil sein, wenn die eingestellte Dosis größer ist als die noch verfügbare.

Falls ein Dosierglied wie bevorzugt zusätzlich zu dem Antriebsglied vorgesehen ist, sind im Kupplungseingriff das Antriebsglied und das Dosierglied vorteilhafterweise voneinander entkoppelt, so dass während der Antriebsbewegung des Antriebsglieds an dem Dosierglied keine auf das Antriebsglied zurückwirkende Manipulationen vorgenommen werden können.

In Ausführungen, in denen das Antriebsglied das Kupplungseingangsglied wie bevorzugt rotatorisch antreibt, kann insbesondere eine Spiralfeder das Antriebsglied bilden. Die Spiralfeder ist um eine Rotationsachse der Rotationsbewegung gewunden, wobei wenigstens eine äußere Federwindung eine innere umgibt. Vorzugsweise weist die Feder bezüglich der Rotationsachse überall die Steigung Null auf. Durch die Verwendung der Spiralfeder kann axiale Baulänge eingespart werden, insbesondere im Vergleich zu Federn, deren Windungen axial nebeneinander angeordnet sind. Die Spiralfeder ist mit einem ihrer beiden Enden, vorzugsweise dem radial inneren Ende, verdrehgesichert mit dem Kupplungseingangsglied verbunden. Das andere Ende, vorzugsweise das radial äußere Ende ist verdrehgesichert mit dem zweiten Gehäuseteil verbunden. Vorteilhafterweise bildet das Kupplungseingangsglied eine Trommel, auf der die Spiralfeder aufgewickelt ist. Bei der Einstellung der Dosis wird das Kupplungseingangsglied um die Rotationsachse gedreht, wodurch die Spiralfeder gespannt wird. Durch eine geeignete Verdrehsperre, beispielsweise eine Ratsche, wird sichergestellt, dass das Kupplungseingangsglied nur in eine Richtung gedreht werden kann. Die Verdrehsperre ist vorzugsweise lösbar, um eine falsch eingestellte Dosis korrigieren zu können. Bei einem Lösen der Verdrehsperre kann sich bei fehlerhafter Bedienung schlimmstenfalls das Kupplungseingangsglied unter der Einwirkung der gespannten Antriebsfeder zu weit zurückdrehen. Da der Kupplungseingriff, der das Kupplungseingangsglied mit der Kolbenstange koppelt, bei der Einstellung der Dosis noch nicht hergestellt ist, da das Kupplungseingangsglied von der Kolbenstange entkoppelt ist, können derartige Fehlbedienungen sich nicht auf die Kolbenstange auswirken.

In besonders bevorzugten Ausführungen, in denen die Kupplung das genannte Kupplungseingangsglied und das genannte Kupplungsausgangsglied umfasst, entkoppelt das Entkopplungsglied in der Entkopplungsposition das Kupplungsausgangsglied von dem Kupplungseingangsglied. Falls der Kupplungseingriff unmittelbar zwischen diesen beiden Kupplungsgliedern besteht, werden diese von dem Entkopplungsglied in der Entkopplungsposition in einer voneinander abgerückten Position gehalten. In bevorzugten Ausführungen umfasst die Kupplung jedoch zusätzlich ein Kupplungszwischenglied, das eingangsseitig mit dem Kupplungseingangsglied und ausgangsseitig mit dem Kupplungsausgangsglied in je einem Eingriff ist und so den Kupplungseingriff herstellt. Für die Entkopplung genügt es vorzugsweise, nur einen dieser beiden Eingriffe des Kupplungszwischenglieds zu lösen. Alternativ löst oder verhindert das Entkopplungsglied in der Entkopplungsposition beide Eingriffe des Kupplungszwischenglieds. Das Kupplungszwischenglied kann das im Zusammenhang mit dem Ruhezustand beschriebene sein.

Bevorzugte Merkmale werden auch in den Unteransprüchen und deren Kombinationen beschrieben.

Obgleich die vorstehend beschriebenen Weiterbildungen und die in den Unteransprüchen beschriebenen Merkmale besonders vorteilhaft den Gegenstand von Anspruch 1 weiterbilden, sind sie vorteilhaft auch für Injektionsgeräte, die zwar die erfmdungsgemäße Kupplung, nicht aber unbedingt das Merkmal der Entkopplung bei einem Wechsel des Behältnisses aufweisen. So kann beispielsweise die Sperrung der Kolbenstange im ausgekuppelten Zustand Gegenstand einer Teilungsanmeldung sein.

Nachfolgend werden Ausführungsbeispiele der Erfindung anhand von Figuren erläutert. An den Ausführungsbeispielen offenbar werdende Merkmale bilden je einzeln und in jeder Merkmalskombination die Gegenstände der Ansprüche und auch die vorstehend beschriebenen Ausführungen vorteilhaft weiter. Es zeigen:
- Figur 1: ein Injektionsgerät eines ersten Ausführungsbeispiels in einer perspektivischen Ansicht,
- Figur 2: das Injektionsgerät mit einer geöffneten Kupplung in einem Längsschnitt,
- Figur 3: das Injektionsgerät mit geschlossener Kupplung,
- Figur 4: ein Detail der Figur 2,
- Figur 5: ein Detail der Figur 3,
- Figur 6: das Injektionsgerät nach Einstellung einer Dosis,
- Figur 7: das Injektionsgerät nach Entleerung eines Reservoirs,
- Figur 8: ein Entkopplungsglied und ein Gehäuseteil des Injektionsgeräts,
- Figur 9: einen distalen Abschnitt des Injektionsgeräts mit verbundenen Gehäuseteilen,
- Figur 10: den distalen Abschnitt während des Lösens der Gehäuseteile
- Figur 11: ein Injektionsgerät eines zweiten Ausführungsbeispiels mit geöffneter Kupplung in einem Längsschnitt,
- Figur 12: das Injektionsgerät des zweiten Ausführungsbeispiels mit geschlossener Kupplung in einem anderen Längsschnitt,
- Figur 13: ein Detail der Figur 11,
- Figur 14: ein Detail der Figur 12,
- Figur 15: das Injektionsgerät des zweiten Ausführungsbeispiels nach Einstellung einer Dosis,
- Figur 16: das Injektionsgerät des zweiten Ausführungsbeispiels nach Entleerung des Reservoirs,
- Figur 17: das Injektionsgerät des zweiten Ausführungsbeispiels mit voneinander gelösten Gehäuseteilen,
- Figur 18: ein Detail der Figur 17,
- Figur 19: ein Injektionsgerät eines dritten Ausführungsbeispiels
- Figur 20: einen proximalen Teil des Injektionsgeräts des dritten Ausführungsbeispiels mit geöffneter Kupplung,
- Figur 21: das Injektionsgerät des dritten Ausführungsbeispiels mit geschlossener Kupplung,
- Figur 22: den proximalen Teil des Injektionsgeräts des dritten Ausführungsbeispiels bei einer Dosiskorrektur,
- Figur 23: den proximalen Teil des Injektionsgeräts des dritten Ausführungsbeispiels nach Entleerung des Reservoirs,
- Figur 24: ein Sperrglied und ein Stoppglied des dritten Ausführungsbeispiels.

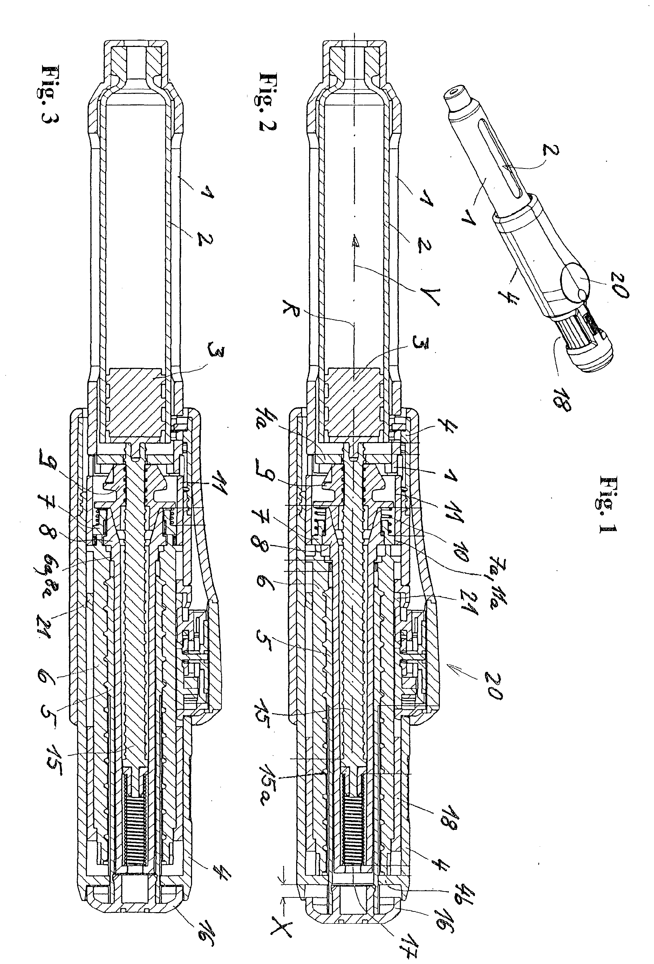

Figur 1 zeigt ein Injektionsgerät eines ersten Ausführungsbeispiels. Das Injektionsgerät weist ein erstes Gehäuseteil 1 und ein zweites Gehäuseteil 4 auf, die lösbar miteinander verbunden sind. Im Ausfuhrungsbeispiel sind die Gehäuseteile 1 und 4 miteinander verschraubt. Das Injektionsgerät ist als schlanker Injektionspen gebildet. Das Gehäuseteil 1 dient der Aufnahme eines mit fluidem Produkt gefüllten Behältnisses 2 und bildet in diesem Sinne ein Reservoir, und das Gehäuseteil 4 dient als Träger für eine Dosier- und Antriebseinrichtung, von der ein Dosierglied 18 zu erkennen ist. Das Gehäuseteil 4 ist im Bereich des Dosierglieds 18 durchbrochen, so dass ein Verwender direkten Zugriff auf das Dosierglied 18 hat. Das Dosierglied 18 ist um eine zentrale Längsachse des Geräts drehbar gelagert und als Hülse gebildet, die bedienerfreundlich an ihrem äußeren Umfang geriffelt ist. Zu erkennen ist ferner eine Dosisanzeige 20, die durch eine Durchbrechung im Mantel des Gehäuseteils 4 seitlich aufgesetzt ist.

Figur 2 zeigt das Injektionsgerät des ersten Ausführungsbeispiels in einem Längsschnitt. In dem Gehäuseteil 1 ist das Behältnis 2 aufgenommen. In dem Behältnis 2 ist ein Kolben 3 in eine Vortriebsrichtung V bewegbar aufgenommen. Der Kolben 3 verschließt das Behältnis 2 an dessen proximalen Ende flüssigkeitsdicht. Durch Vortrieb des Kolbens 3 in die Vortriebsrichtung V wird Produkt durch einen Auslass des Behältnisses 2 verdrängt und ausgeschüttet, vorzugsweise durch eine in den Auslass ragende, mittels eines Nadelhalters an dem distalen Ende des Gehäuseteils 1 befestigten Injektionsnadel. Das Behältnis 2 ist in der Art üblicher Ampullen gebildet. Das Gehäuseteil 1 bildet unmittelbar einen Behältnishalter, im Ausführungsbeispiel einen Ampullenhalter. Das Gehäuseteil 1 ragt mit seinem proximalen Ende in das Gehäuseteil 4 hinein, und ist mit dem Gehäuseteil 4 verschraubt.

Das Gehäuseteil 4 nimmt eine Kolbenstange 15 und die Dosier- und Antriebseinrichtung auf, die als Dosier- und Antriebsmechanik gebildet ist. Die Dosier- und Antriebseinrichtung umfasst in einem Dosier- und Antriebsstrang ein Antriebsglied 5 und eine Kupplung 6-11, die in einem eingekuppelten Zustand, d.h. in einem Kupplungseingriff, das Antriebsglied 5 mit der Kolbenstange 15 koppelt. Die Kolbenstange 15 bildet mit dem Kolben 3 eine Fördereinrichtung. Im eingekuppelten Zustand übertragen Kupplungsglieder 6-10 eine auf das Antriebsglied 5 ausgeübte Antriebskraft auf die Kolbenstange 15. In Figur 2 besteht kein Kupplungseingriff, so dass die Kolbenstange 15 von dem Antriebsglied 5 entkoppelt ist. In diesem ausgekuppelten Zustand kann der Benutzer durch eine Dosierbewegung des Dosierglieds 18, im Ausführungsbeispiel eine Drehbewegung, die zu verabreichende Produktdosis einstellen.

Das Antriebsglied 5 ist hülsenförmig. Es weist an seiner Mantelaußenfläche ein Gewinde um eine in die Vortriebsrichtung V weisende Gewindeachse R auf. Mit diesem Gewinde steht das Antriebsglied 5 in einem Gewindeeingriff mit einem Kupplungseingangsglied 6. Das Kupplungseingangsglied 6 ist ebenfalls hülsenförmig und für den Gewindeeingriff mit einem korrespondierenden Innengewinde versehen. Die Gewindesteigung im Gewindeeingriff ist so groß, dass eine Selbsthemmung nicht eintreten kann. Das Dosierglied 18 umgibt das Kupplungseingangsglied 6 und ist mit dem Kupplungseingangsglied 6 verdrehgesichert und axial nicht beweglich verbunden. Die Kolbenstange 15 ragt in das Antriebsglied 5 und das Kupplungseingangsglied 6.

Die Kolbenstange 15 ist über ihre axiale Länge mit einem Außengewinde versehen. Mit dem Außengewinde ist sie in einem Gewindeeingriff mit einem Kupplungsausgangsglied 9, das mit einem korrespondierenden Innengewinde versehen ist. Auch diese beiden Gewinde weisen eine Steigung auf, die eine Selbsthemmung im Gewindeeingriff verhindert. Die Gewindesteigung ist vorzugsweise geringer als die Gewindesteigung im Gewindeeingriff zwischen dem Antriebsglied 5 und dem Kupplungseingangsglied 6. Eine Kupplungshülse 8 ist mit dem Kupplungsausgangsglied 9 verdrehgesichert und axial nicht beweglich verbunden. Die Kupplungshülse 8 und das Kupplungsausgangsglied 9 können bezüglich der zwischen dem Antriebsglied 5 und der Kolbenstange 15 stattfindenden Bewegungen wie ein einstückiges Bauteil angesehen werden; sie sind zur Aufnahme einer Ausgleichsfeder 17 jedoch zweiteilig ausgeführt und fest miteinander verbunden. Das Kupplungsausgangsglied 9 und die Kupplungshülse 8 sind um die Gewindeachse R des Kupplungsausgangsglieds 9 drehbar, aber axial nicht bewegbar im Gehäuseteil 4 gelagert. Die Kolbenstange 15 durchragt im Gewindeeingriff des Kupplungsausgangsglied 9 und ragt in die Kupplungshülse 8 hinein. Zwischen einem proximalen Ende der Kupplungshülse 8 und einem proximalen Ende der Kolbenstange 15 ist die Ausgleichsfeder 17 eingespannt, die als Druckfeder in Vortriebsrichtung V auf die Kolbenstange 15 wirkt. Die Ausgleichsfeder 17 drückt über einen drehbar an der Kolbenstange 15 abgestützten Teller 15a, den ein Flansch einer auf die Kolbenstange 15 gesetzten Hülse bildet, auf die Kolbenstange 15.

Die Kolbenstange 15 ist in einer Linearführung 4a relativ zu dem Gehäuseteil 1 nicht verdrehbar in und gegen die Vortriebsrichtung V linear geführt. Das Antriebsglied 5 ist relativ zu dem Gehäuseteil 4 ebenfalls in und gegen die Vortriebsrichtung V bewegbar lineargeführt, wofür das Gehäuseteil 4 unmittelbar eine Linearführung 4b bildet.

Die Gewindeachse der Kolbenstange 15 bildet die Hauptbewegungsachse der Vorrichtung. Für die rotatorische Antriebsbewegung des Kupplungseingangsglieds 6 und über das Kupplungszwischenglied 7 auch des Kupplungsausgangsglieds 9 bildet sie eine Rotationsachse R. Sie bildet beide Gewindeachsen. Desweiteren bildet sie die Translationsachse für die Kolbenstange 15 und das Antriebsglied 5.

Die Kupplung 6-11 umfasst ferner ein Kupplungszwischenglied 7 und ein Rückstellglied 10, das als Druckfeder gebildet ist, und das Kupplungszwischenglied 7 mit einer gegen die Vortriebsrichtung V wirkenden Elastizitätskraft beaufschlagt. Das Rückstellglied 10 ist zwischen dem Kupplungsausgangsglied 9 und dem Kupplungszwischenglied 7 eingespannt.

Falls auf das Antriebsglied 5 keine in die Vortriebsrichtung V wirkende Kraft ausgeübt wird, sorgt das Rückstellglied 10 über das Kupplungszwischenglied 7 dafür, dass der Kupplungseingriff gelöst ist. Diesen Zustand zeigt die Figur 2. Das Kupplungseingangsglied 6 ist in die Vortriebsrichtung V auf Anschlag gegen das Kupplungszwischenglied 7 und wird von dem Rückstellglied 10 über das Kupplungszwischenglied 7 in eine proximale Endposition gedrückt. Das Rückstellglied 10 hält das Kupplungseingangsglied 6 relativ zu dem Kupplungsausgangsglied 9 und der daran befestigten Kupplungshülse 8 mittels des Kupplungszwischenglieds in einer Halteposition. Das Rückstellglied 10 und das Kupplungszwischenglied 7 bilden somit eine kraftschlüssig wirkende Halteeinrichtung für das Kupplungseingangsglied 6.

Figur 3 zeigt das Injektionsgerät in einem gekoppelten Zustand. Zwischen dem Kupplungseingangsglied 6 und der Kupplungshülse 8 besteht ein Kupplungseingriff. Für den Kupplungseingriff bilden das Kupplungseingangsglied 6 und die Kupplungshülse 8 Eingriffselemente, die im Kupplungseingriff eine verdrehgesicherte Verbindung der beiden Glieder 6 und 8 um die in die Vortriebsrichtung V weisende Gewindeachse R herstellen. Die Eingriffselemente wirken als Nuten und Federn oder Verzahnungen zusammen, die zur Vortriebsrichtung V parallel und um die Gewindeachse R gleichmäßig verteilt gebildet sind.

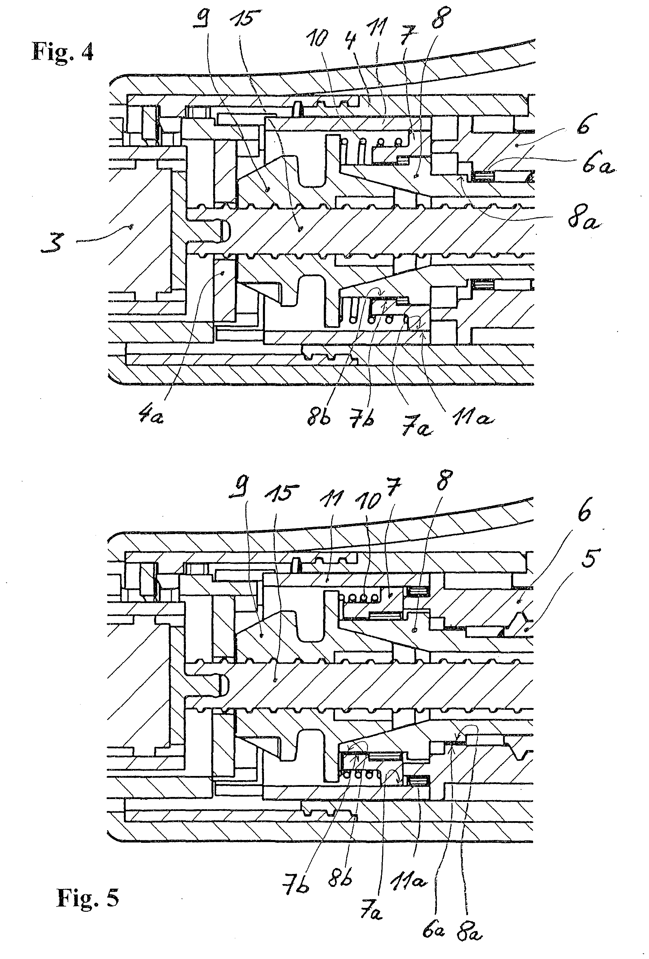

Die Figuren 4 und 5 zeigen den Bereich des Kupplungseingriffs im Detail. Figur 4 zeigt das Gerät in dem entkoppelten und Figur 5 in dem gekoppelten Zustand. Figur 4 korrespondiert somit mit Figur 2, und Figur 5 korrespondiert mit Figur 3.
Im entkoppelten Zustand ist das Kupplungseingangsglied 6 entgegen der Vortriebsrichtung V von der Kupplungshülse 8 abgerückt, so dass das Kupplungseingangsglied 6 relativ zu der Kupplungshülse 8 und dem damit fest verbundenen Kupplungsausgangsglied 9 frei drehbar ist. Gleichzeitig ist das Kupplungsausgangsglied 9 über die Kupplungshülse 8, das Kupplungszwischenglied 7 und ein Entkopplungsglied 11 verdrehgesichert mit dem Gehäuseteil 4 verbunden. Für diese verdrehsichere Kopplung sind das Kupplungszwischenglied 7 an einer der Kupplungshülse 8 radial zugewandten Innenfläche mit Eingriffselementen 7b und die Kupplungshülse 8 mit korrespondierenden Eingriffselementen 8b versehen. Für den verdrehgesicherten Eingriff mit dem Entkopplungsglied 11 sind das Kupplungszwischenglied 7 an einer äußeren Umfangsfläche mit Eingriffselementen 7a und das Entkopplungsglied 11 an einer Mantelinnenfläche mit radial zugewandten Eingriffselementen 11a versehen, die im entkoppelten Zustand wie die Eingriffselemente 7b und 8b in der Art von Nuten und Federn oder Verzahnungen, die zur Vortriebsrichtung V parallel sind, ineinander greifen. Das Kupplungszwischenglied 7 ist in seinem verdrehgesicherten Eingriff mit der Kupplungshülse 8 und seinem verdrehgesicherten Eingriff mit dem Entkopplungsglied 11 in und gegen die Vortriebsrichtung V axial bewegbar, wobei sich der Eingriff mit dem Entkopplungsglied 11 bei einer Bewegung in die Vortriebsrichtung V löst.

Wird das Antriebsglied 5 betätigt, indem auf ein Auslöseelement 16 in die Vortriebsrichtung V eine Druckkraft ausgeübt wird, vollführen das Antriebsglied 5 und das Kupplungseingangsglied 6 gemeinsam einen axialen Kupplungshub der Länge X. Bei dieser Antriebshubbewegung bzw. Kupplungsbewegung stößt das Kupplungseingangsglied 6 das Kupplungszwischenglied 7 gegen die rückstellende Elastizitätskraft des Rückstellglieds 10 in die Vortriebsrichtung V. Im Verlaufe der Hubbewegung gelangen die Eingriffselemente 6a und 8a miteinander in Eingriff, während das Kupplungszwischenglied 7 sich gleichzeitig relativ zu dem Entkopplungsglied 11 bewegt bis es aus dem verdrehgesicherten Eingriff mit dem Entkopplungsglied 11 gelangt. Dabei bleibt das Kupplungszwischenglied 7 in dem verdrehgesicherten Eingriff mit der Kupplungshülse 8. Die Kupplungsbewegung wird durch einen Anschlag des Auslöseelements 16 an der Kupplungshülse 8, im Ausführungsbeispiel an deren proximalen Stirnfläche, begrenzt (Figur 3).
Figur 5 zeigt das Injektionsgerät im gekoppelten Zustand. Die Eingriffselemente 6a und 8a sind in axialer Überdeckung, so dass der Kupplungseingriff als verdrehgesicherter Eingriff zwischen dem Kupplungseingangsglied 6 und der Kupplungshülse 8 hergestellt ist. Der Eingriff zwischen dem Kupplungszwischenglied 7 und dem Entkopplungsglied 11 löst sich erst, nachdem der Kupplungseingriff sicher hergestellt ist.

Für die Einstellung der Dosis verdreht der Verwender das Dosierglied 18, das dabei in leicht lösbaren Rastpositionen verrastet. Das Dosierglied 18 ist verdrehgesichert und auch axial unbeweglich mit dem Kupplungseingangsglied 6 verbunden, so dass dieses mitdreht. Durch diese Dosierbewegung des Kupplungseingangsglieds 6 wird das bei 4b in und gegen die Vortriebsrichtung V linear geführte Antriebsglied 5 in die proximale Richtung bewegt und ragt dann aus dem Gehäuseteil 4. Der axiale Dosierweg des Antriebsglieds 5 ergibt sich aus dem Drehwinkel, um den das Dosierglied 18 verdreht wird und der Gewindesteigung im Gewindeeingriff zwischen dem Antriebsglied 5 und dem Kupplungseingangsglied 6, das in die Vortriebsrichtung V gegen das Kupplungszwischenglied 7 und gegen die Vortriebsrichtung V gegen das Gehäuseteil 4 auf Anschlag ist.

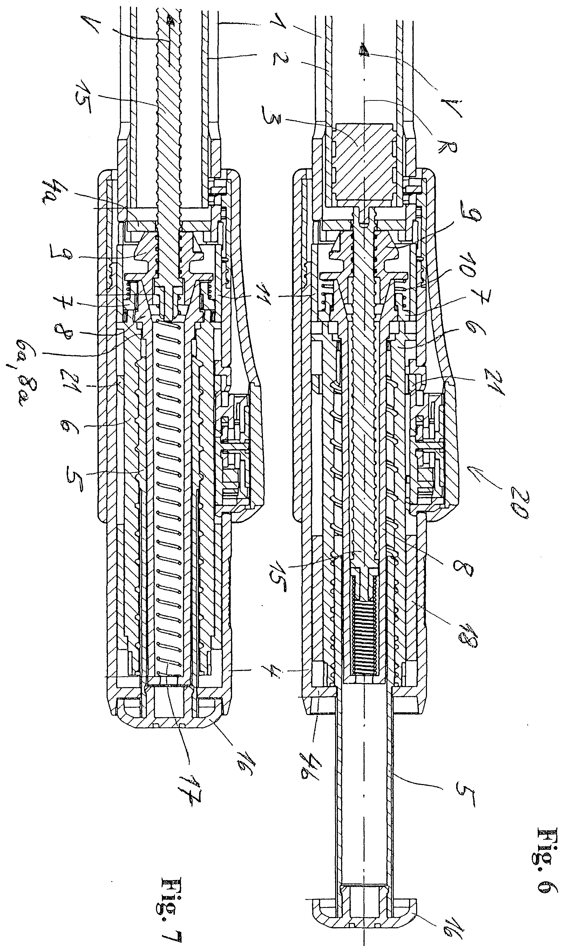

Figur 6 zeigt das Injektionsgerät mit noch vollständig gefülltem Behältnis 2 nach dem Einstellen einer ersten Dosis. In diesem Zustand durchsticht der Benutzer mit der Injektionsnadel die Haut für eine subkutane Injektion. Nachdem die Injektionsnadel platziert ist, betätigt der Benutzer das Antriebsglied 5, indem er dieses in die Vortriebsrichtung V in das Gehäuseteil 4 hinein drückt. In dem ersten Abschnitt der Antriebsbewegung, der Kupplungsbewegung bzw. dem Kupplungshub X, nimmt das Antriebsglied 5 das Kupplungseingangsglied 6 gegen die elastische Rückstellkraft des Rückstelldements 10 mit bis der Kupplungseingriff mit der Kupplungshülse 8 hergestellt und der verdrehgesicherte Eingriff zwischen dem Kupplungszwischenglied 7 und dem Entkopplungsglied 11 gelöst sind. Sobald die Kupplungshülse 8 und damit gemeinsam das Kupplungsausgangsglied 9 um die gemeinsame Rotationsachse R frei drehen können, ist der Kupplungshub X beendet, und es schließt sich als zweiter Abschnitt der Antriebsbewegung ein Ausschütthub an. Während des Ausschütthubs wird das Antriebsglied 5 weiter in die Vortriebsrichtung V gedrückt. Da das Kupplungseingangsglied 6 über den axialen Anschlag gegen das Kupplungszwischenglied 7 keine weitere Bewegung in die Vortriebsrichtung V ausführen kann, dreht es sich in dem Gewindeeingriff mit dem verdrehgesichert geführten Antriebsglied 5 um die gemeinsame Gewindeachse R. Das Kupplungseingangsglied 6 nimmt bei seiner Drehbewegung im Kupplungseingriff die Kupplungshülse 8 und diese das Kupplungsausgangsglied 9 mit. Die Kupplungshülse 8 ist gemeinsam mit dem Kupplungsausgangsglied 9 axial nicht bewegbar in dem Gehäuseteil 4 gehalten. Die Drehbewegung des Kupplungsausgangsglieds 9 bewirkt über den Gewindeeingriff mit der Kolbenstange 15 und deren verdrehgesicherten Linearführung bei 4a den Vortrieb und somit die Ausschüttbewegung der Kolbenstange 15 und damit gemeinsam des Kolbens 3. Sobald der Injektionsknopf 16 im Zuge der Antriebs- und Ausschüttbewegung in Anschlagkontakt gegen die Kupplungshülse 8 gelangt (Figur 3), ist der Ausschüttvorgang beendet.

Nimmt der Benutzer den Druck von dem Auslöseelement 16, so bewegt das Rückstellglied 10 über das Kupplungszwischenglied 7 das Kupplungseingangsglied 6 wieder in die aus dem Kupplungseingriff abgerückte Halteposition, wie sie in den Figuren 2 und 4 dargestellt ist. Das Kupplungseingangsglied 6 und damit gemeinsam das Antriebsglied 5, das Dosierglied 18 und die Dosisanzeige 20 werden durch die Ausrückbewegung des Kupplungseingangsglieds 6 von dem Kupplungsausgangsglied 9 und somit von der Kolbenstange 15 entkoppelt. Andererseits wird die Kolbenstange 15 über das zurückfahrende Kupplungszwischenglied 7 und das Entkopplungsglied 11 wieder verdrehgesichert mit dem Gehäuseteil 4 verbunden.

Figur 7 zeigt das Injektionsgerät am Ende einer letzten Ausschüttung, mit der das Behältnis 2 entleert wurde.

Für einen Austausch des entleerten Behältnisses 2 wird das Gehäuseteil 1 von dem Gehäuseteil 4 gelöst, im Ausführungsbeispiel durch eine Schraubbewegung. Bei dem Lösen der Gehäuseteile 1 und 2 wird das Entkopplungsglied 11 automatisch relativ zu dem Gehäuseteil 4 gegen die Richtung der Kupplungsbewegung des Kupplungseingangsglieds 6, im Ausführungsbeispiel gegen die Vortriebsrichtung V, bewegt. Das Gehäuseteil 4 lagert das Entkopplungsglied 11 entsprechend. Der von dem Entkopplungsglied 11 relativ zu dem Gehäuseteil 4 dabei zurückgelegte axiale Weg ist so lang wie der Kupplungshub X, so dass das Entkopplungsglied 11 nach dem Lösen der Gehäuseteile 1 und 4 dem Kupplungseingangsglied 6 axial gegenüberliegend dieses blockiert und das Kupplungseingangsglied 6 nicht mehr in die Vortriebsrichtung V bewegt werden kann, zumindest nicht in den Kupplungseingriff mit der Kupplungshülse 8. Die Blockierung des Kupplungseingangsglieds 6 in der ausgerückten Position verhindert, dass das Kupplungsausgangsglied 9 in eine verdrehgesicherte Verbindung mit dem Gehäuseteil 4 gelangen und dadurch ein Zurückschieben der Kolbenstange 15 verhindern kann. Es wird mit anderen Worten sichergestellt, dass die Kolbenstange 15 blockierfrei in das Gehäuseteil 4 zurückgeschoben werden kann.

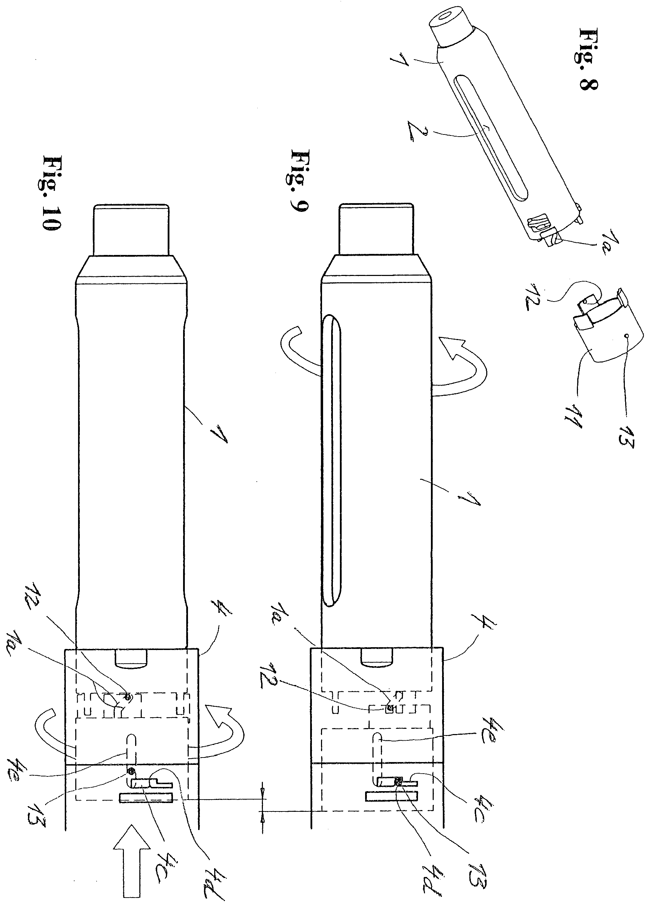

Figur 8 zeigt das Entkopplungsglied 11 und das erste Gehäuseteil 1 in einer perspektivischen Ansicht. Das Entkopplungsglied 11 ist ein Hülsenteil und weist in einem distalen Abschnitt drei nach radial einwärts ragende Eingriffselemente 12 und in einem proximalen Abschnitt ein nach radial auswärts ragendes Fixierelement 13 auf.

Figur 9 zeigt das Gehäuseteil 1 und einen Verbindungsabschnitt des Gehäuseteils 4, wobei das verdeckte Entkopplungsglied 11 strichliert dargestellt ist. Für seine Entkopplungsfunktion ist das Entkopplungsglied 11 in dem Verbindungsabschnitt des Gehäuseteils 4 drehbar und axial bewegbar aufgenommen. Die relative Bewegbarkeit wird durch eine Axialführung 4e und eine Umfangsführung 4c bestimmt, entlang denen das Eingriffselement 13 nacheinander entlangfährt, wenn das Gehäuseteil 1 von dem Gehäuseteil 4 gelöst wird. Die Umfangsführung 4c erstreckt sich rechtwinklig zur Axialführung 4e in Umfangsrichtung um die Schraubachse. Sie ist als Durchbruch oder Ausnehmung im Gehäuseteil 4 gebildet.

Mit dem Gehäuseteil 1 ist das Entkopplungsglied 11 in einem Führungseingriff. Für den Führungseingriff ist an einer Mantelaußenfläche des Gehäuseteils 1 pro Eingriffselement 12 eine Führungskurve 1a gebildet, die das Eingriffselement 12 und somit das Entkopplungsglied 11 bei dem Lösen der Gehäuseteile 1 und 4 führt. Eine parallel beabstandete weitere Führungskurve 1a führt das Entkopplungsglied 11 bei einem Verbinden der Gehäuseteile 1 und 4 entsprechend (Fig. 10). Die Führungskurve la verläuft in einem distalen Abschnitt schräg, d.h. mit einer Steigung, zu der Schraubachse der Schraubverbindung der Gehäuseteile 1 und 4, so dass bei der für das Lösen erforderlichen Relativdrehung zwischen den Gehäuseteilen 1 und 4 das Eingriffselement 12 an der Führungskurve 1a entlang gleitend eine Axialbewegung des Entkopplungsglieds 11 relativ zu dem Gehäuseteil 4 gegen die Vortriebsrichtung V ausführt, bis das Fixierelement 13 auf die axiale Höhe der Umfangsführung 4c gelangt. Die Steigung beträgt etwa 45° und ist konstant. Grundsätzlich kann sie aus dem gesamten Bereich größer 0° und kleiner 180° gewählt werden und gegebenenfalls auch veränderlich sein, solange die zum Lösen der Gehäuseteile 1 und 4 erforderliche Relativbewegung, im Ausführungsbeispiel eine Schraubbewegung, eine Bewegung des Entkopplungsglieds entgegen der vom Kupplungseingangsglied zum Einkuppeln auszuführenden Kupplungsbewegung X bewirkt. Ein distaler Abschnitt der Führungskurve 1a verläuft axial, so dass bei dem weiteren Auseinanderschrauben der Gehäuseteile 1 und 4 das Fixierelement 13 längs der Umfangsführung 4c bewegt wird. Im Verlaufe dieser relativen Umfangsbewegung zwischen dem Entkopplungsglied 11 und dem Gehäuseteil 4 gleitet das Fixierelement 13 über ein Fixierelement 4d im Bereich der Umfangsführung 4c. Das Fixierelement 4d ist als Nocken an einem Streifenabschnitt des Gehäuseteils 4 gebildet. Der Streifenabschnitt wirkt als beidseits fest eingespannte Biegefeder, die bei der Bewegung des Fixierelements 13 über das Fixierelement 4d federnd nachgibt, um anschließend wieder in ihre Ausgangslage zurückzufedern und einen lösbaren Rasteingriff für das Entkopplungsglied 11 zu bilden. In der Rastposition ist das Fixierelement 13 in die eine Umfangsrichtung an dem Fixierelement 4d und in die andere Umfangsrichtung an einer in der Umfangsführung 4c gebildeten Schulter auf Anschlag und dadurch in beide Umfangsrichtungen fixiert.

Figur 10 zeigt die beiden Gehäuseteile 1 und 4 und das Entkopplungsglied 11 nachdem dessen Fixierelement 13 hinter das Fixierelement 4d des Gehäuseteils 4 bewegt worden ist. Das Entkopplungsglied 11 ist über die Fixierelemente 4d und 13 mit dem Gehäuseteil 4 in dem lösbaren Rasteingriff und auf diese Weise an dem Gehäuseteil 4 verdrehgesichert und axial fixiert. In der in Figur 10 gezeigten Rastposition blockiert das Entkopplungsglied 11 das Kupplungseingangsglied 6 und stellt dadurch die Entkopplung von Antriebsglied 5 und Kolbenstange 15 sicher. Sobald das Entkopplungsglied 11 die Rastposition erreicht hat, bewegt sich dessen Eingriffselement 12 bei dem weiteren Auseinanderschrauben der Gehäuseteile 1 und 4 aus dem Führungseingriff mit der Führungskurve 1a. Die Führungskurve 1a ist entsprechend geformt.

Bei einem erneuten Zusammenschrauben der Gehäuseteile 1 und 4 zentrieren sich diese bezüglich der Umfangsrichtung mittels zusammenwirkender Zentrierelemente, so dass das Eingriffselement 12 des Entkopplungsglieds 11 wieder in Eingriff mit der Führungskurve 1a gelangt. Sobald der Führungseingriff hergestellt ist wird bei dem weiteren Zusammenschrauben das Entkopplungsglied 11 automatisch aus dem Rasteingriff der Fixierelemente 4d und 13 bewegt, bis es relativ zu dem Gehäuseteil 4 wieder die gleiche Position wie in Figur 9 und den Figuren 2 bis 7 einnimmt; dies entspricht der Betriebsposition des Entkopplungsglieds 11.

Bei oder vor dem Zusammenschrauben wird die Kolbenstange 15 einfach in das Gehäuseteil 4 zurückgeschoben, was wegen des gelösten Kupplungseingriffs eine Drehbewegung des Kupplungsausgangsglieds 9 bewirkt.

Die Dosisanzeige 20 des ersten Ausführungsbeispiels ist über ein Anzeigekopplungsglied 21 und das Kupplungseingangsglied 6 mit dem Antriebsglied 5 gekoppelt. Das Anzeigekopplungsglied 21 ist mit dem Kupplungseingangsglied 6 verdrehgesichert verbunden, indem es im Ausführungsbeispiel einen Ring bildend auf dem Kupplungsglied 6 und relativ zu diesem in und gegen die Richtung der Kupplungsbewegung X bewegbar ist. Das Anzeigekopplungsglied 21 ist umgekehrt zu dem Gehäuseteil 4 um die Rotationsachse R drehbar, wird relativ zu dem Gehäuseteil 4 jedoch axial nicht bewegbar gehalten. Das Anzeigekopplungsglied 21 weist umlaufend eine Verzahnung auf, die im Ausführungsbeispiel als Kegelverzahnung gebildet ist, mit der es mit einem Getriebe der Dosisanzeige 20 in Zahneingriff ist, um die Dosierbewegung und auch die Antriebsbewegung in das Getriebe einzuleiten.

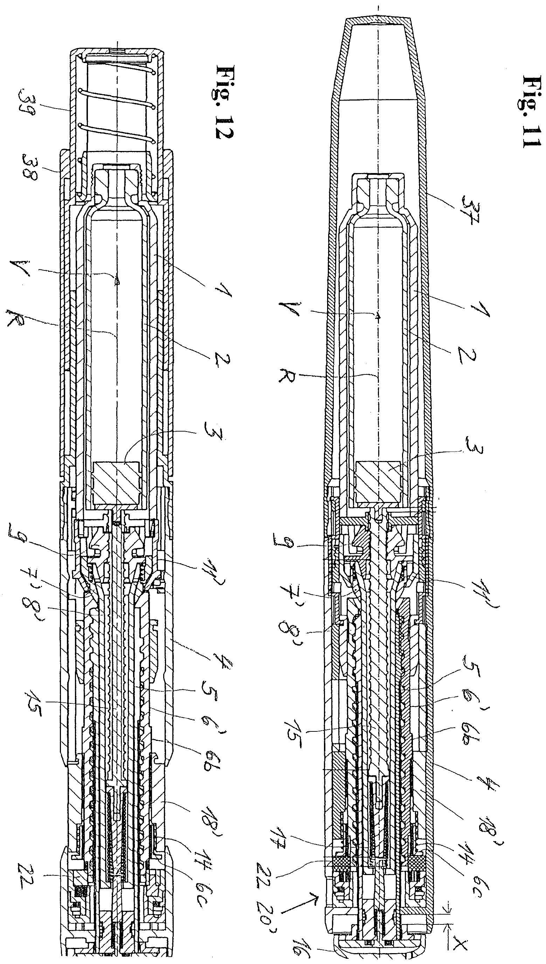

Die Figuren 11 bis 18 zeigen ein Injektionsgerät eines zweiten Ausführungsbeispiels. Das Injektionsgerät des zweiten Ausführungsbeispiels weist gegenüber dem Gerät des ersten Ausführungsbeispiels einige Modifikationen hinsichtlich der Kopplung und Entkopplung von Antriebsglied 5 und Kolbenstange 15 auf. Das Antriebsglied 5 und die Kolbenstange 15 selbst und auch das grundsätzliche Zusammenwirken beim Koppeln und Entkoppeln sind jedoch gleich geblieben. Die der Funktion nach gleichen Komponenten sind mit den gleichen Bezugsziffern wie im ersten Ausführungsbeispiel versehen. Um auf Modifikationen hinzuweisen, sind die betreffenden Komponenten mit den gleichen, aber apostrophierten Bezugsziffern versehen.

Figur 11 zeigt das Injektionsgerät im Ruhezustand, in dem das Antriebsglied 5 von der Kolbenstange 15 entkoppelt ist. Das erste Gehäuseteil 1 ist mit einer mit dem Gehäuseteil 4 verbundenen Schutzkappe 37 abgedeckt, die für die Produktverabreichung abgenommen wird. Im Unterschied zu dem ersten Ausführungsbeispiel wird der Kupplungseingriff zwischen dem modifizierten Kupplungseingangsglied 6' und dem modifizierten Kupplungszwischenglied 7' hergestellt und gelöst.

Figur 12 zeigt das Injektionsgerät des zweiten Ausführungsbeispiels im gekoppelten Zustand, der durch die Beaufschlagung des Auslöseelements 16 und damit des Antriebsglieds 5 und des Kupplungseingangsglieds 6' durch eine in die Vortriebsrichtung V wirkende Antriebskraft hergestellt wird. Allerdings wurde wie bereits bei der korrespondierenden Figur 3 des ersten Ausführungsbeispiels noch keine Dosis oder zum Primen nur eine geringe Dosis von wenigen Einheiten ausgewählt. Die Schutzkappe 37 wurde durch ein Gehäuseteil 38 ersetzt, das auf das Gehäuseteil 4 aufgesetzt und mit diesem verschnappt ist. Das Gehäuseteil 38 lagert einen Nadelschutz 39, vorzugsweise in Form einer Nadelschutzhülse, gegen die Vortriebsrichtung V federnd beweglich. Bei einem Einstechen der nicht dargestellten Injektionsnadel federt der Nadelschutz 39 gegen die Vortriebsrichtung V in das Gehäuseteil 38 ein; in Umkehrung dieser Bewegung sticht die Nadel durch eine distale Öffnung des Nadelschutzes 39 vor.

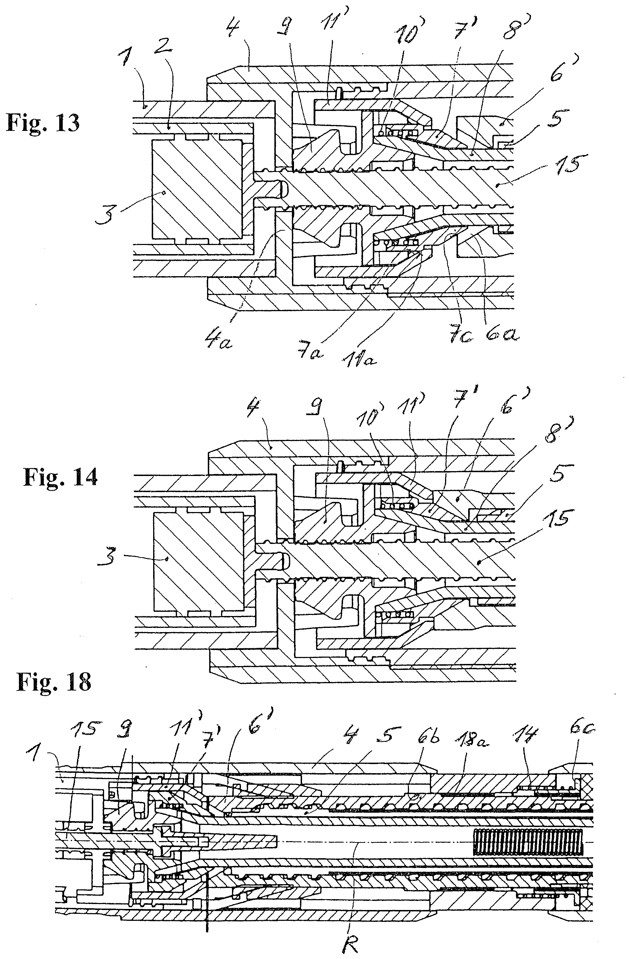

Die Figuren 13 und 14 zeigen den Bereich des Kupplungseingriffs im Detail, wobei Figur 13 für den entkoppelten Zustand und Figur 14 den gekoppelten Zustand steht. Im Unterschied zum ersten Ausführungsbeispiel weisen die Eingriffselemente 6a und 7c, zwischen denen der Kupplungseingriff hergestellt wird, zu der Vortriebsrichtung V eine Neigung auf. Im Ausführungsbeispiel sind die Eingriffselemente 6a und 7c je in der Art eines um die Gewindeachse der Kolbenstange 15 umlaufenden Kegelzahnkranzes gebildet. Das Kupplungseingangsglied 6' bildet dabei seine Eingriffselemente 6a an seinem distalen Ende als Innenkonus, und das Kupplungszwischenglied 7' bildet die Eingriffselemente 7c an seinem proximalen Ende als Außenkonus. Die konischen Eingriffsflächen sind zueinander kongruent und liegen einander axial zugewandt mit dem lichten Abstand X unmittelbar gegenüber. Die Kupplungsflächen könnten statt konisch auch kongruent konvex/konkav geformt sein.

Das Kupplungszwischenglied 7' ist im Unterschied zum ersten Ausführungsbeispiel axial bewegbar und in jeder Axialposition verdrehgesichert mit dem Kupplungsausgangsglied 9 in einem Eingriff. Es ist wieder als Hülsenteil gebildet und axial gleitverschiebbar auf dem Kupplungsausgangsglied 9 gelagert. Hierfür durchgreift es die axial entsprechend geschlitzte Kupplungshülse 8', was in den Figuren allerdings nicht sichtbar ist. Die verdrehgesicherte Verbindung wird formschlüssig über Eingriffselemente geschaffen, die als axial gerade Verzahnungen gebildet sind. Das nach Ausbildung und Einbau gleiche, hinsichtlich seiner Funktion jedoch reduzierte Rückstellglied 10' ist wie im ersten Ausführungsbeispiel zwischen dem Kupplungsausgangsglied 9 und dem Kupplungszwischenglied 7' gespannt und beaufschlagt letzteres gegen die Vortriebsrichtung V mit einer Elastizitätskraft. Im entkoppelten Zustand, in dem das Kupplungseingangsglied 6', wie in Figur 13 gezeigt, gegen die Vortriebsrichtung V von dem Kupplungszwischenglied 7' abgerückt ist, drückt das Rückstellglied 10' das Kupplungszwischenglied 7' in den verdrehgesicherten Eingriff mit dem Entkopplungsglied 11'. Die entsprechenden Eingriffselemente sind wieder mit 7a und 11a bezeichnet. Auch die Eingriffselemente 7a und 11a sind als kegelige Zahnkränze gebildet. Der Eingriff von Kupplungszwischenglied 7' und Entkopplungsglied 11' kann alternativ rein reibschlüssig sein. Die Eingriffselemente 7a und 11a weisen in diesem Fall einander zugewandte, kongruente Reibflächen auf, im Ausführungsbeispiel wären dies die einander zugewandten Konusflächen.

Eine weitere Modifikation besteht bei dem Dosierglied 18'. Im Unterschied zu dem Dosierglied 18 des ersten Ausführungsbeispiels ist das Dosierglied 18' relativ zu dem Gehäuseteil 4 in Richtung der Kupplungsbewegung X, im Ausführungsbeispiel die axiale Richtung, nicht bewegbar. Stattdessen ist das Kupplungseingangsglied 6' zwar wieder verdrehgesichert, aber axial beweglich mit dem Dosierglied 18' verbunden. Der verdrehgesicherte Eingriff zwischen dem Kupplungseingangsglied 6' und dem Dosierglied 18' besteht im entkoppelten Zustand von Antriebsglied 5 und Kolbenstange 15 und wird im Verlaufe des Kupplungshubs X gelöst, nämlich unmittelbar bevor die verdrehgesicherte Verbindung zwischen dem Kupplungsausgangsglied 9 und dem Gehäuseteil 4 gelöst wird. Für diesen Eingriff sind das Kupplungseingangsglied 6' und das Dosierglied 18' mit Eingriffselementen 6b und 18a versehen, die an radial einander zugewandten Mantelflächen der beiden Glieder 6' und 18' in der Art von Nuten und Federn gebildet sind. Bezüglich der verdrehgesicherten Verbindung zwischen dem Kupplungseingangsglied 6' und dem Dosierglied 18' sei auch auf die Figuren 11 und 12 verwiesen. In dem in Figur 11 dargestellten entkoppelten Zustand besteht die verdrehgesicherte Verbindung, und in dem in Figur 12 dargestellten gekoppelten Zustand ist sie gelöst.

Noch ein Unterschied zum ersten Ausführungsbeispiel besteht hinsichtlich der Halteeinrichtung. Das Rückstellglied 10' hat im zweiten Ausführungsbeispiel keine, die Kupplungsglieder 6' und 9 voneinander trennende Wirkung. Die Halteeinrichtung des zweiten Ausführungsbeispiels umfasst ein Rückstellglied 14, ein Stützstruktur 6c und das Dosierglied 18'. Das Rückstellglied 14 beaufschlagt das Kupplungseingangsglied 6' über das Stützstruktur 6c mit einer elastischen Rückstellkraft, die der Kupplungsbewegung X des Kupplungseingangsglieds 6' entgegenwirkt. In Richtung der Kupplungsbewegung X, die in den Ausführungsbeispielen mit der Vortriebsrichtung V zusammenfällt, ist das Rückstellglied 14 an dem Dosierglied 18' abgestützt, das hierfür eine Abstützschulter bildet. Das Stützstruktur 6c ist in und gegen die Richtung der Kupplungsbewegung X unbeweglich mit dem Kupplungseingangsglied 6' verbunden. Es ist als kurze Hülse mit einem Außenflansch gebildet, an dem sich das Rückstellglied 14 abstützt. Gegen die Richtung der Kupplungsbewegung X liegt das Stützstruktur 6c in Bezug auf das Gehäuseteil 4 auf Anschlag. Durch die Kupplungsbewegung X wird das Kupplungseingangsglied 6' gegen die elastische Rückstellkraft des Rückstellglieds 14 in den Kupplungseingriff mit dem Kupplungszwischenglied 7' bewegt. Das Rückstellglied 14 ist wie im ersten Ausführungsbeispiel als eine in Richtung der Kupplungsbewegung X mit einer Druckkraft beaufschlagte Druckfeder gebildet.

Die Arbeitsweise der modifizierten Kupplung 6'-11', 14' ist die gleiche wie die Kupplung des ersten Ausführungsbeispiels. So ist das Kupplungsausgangsglied 9 in dem entkoppelten Zustand über die Kupplungshülse 8', das Kupplungszwischenglied 7' und das Entkopplungsglied 11' verdrehgesichert mit dem Gehäuseteil 4 verbunden. Durch Betätigung des Injektionsknopfs 16 und damit einhergehend der Ausführung des Kupplungshubs X (Figur 11) wird der Kupplungseingriff hergestellt, im zweiten Ausführungsbeispiel zwischen dem Kupplungseingangsglied 6' und dem Kupplungszwischenglied 7'. In der ersten Phase des Kupplungshubs X greifen die Eingriffselemente 6a und 7c ineinander, so dass das Kupplungseingangsglied 6' über das Kupplungszwischenglied 7' und die Kupplungshülse 8' verdrehgesichert mit dem Kupplungsausgangsglied 9 verbunden ist. Erst nach Herstellung des verdrehgesicherten Eingriffs wird durch das in die Vortriebsrichtung V drückende Kupplungseingangsglied 6' das Kupplungsglied 7' aus dem Eingriff mit dem Entkopplungsglied 11' bewegt, so dass das Kupplungsausgangsglied 9 frei um die mit der Kolbenstange 15 gebildete Gewindeachse R drehen kann und der Kupplungseingriff vollständig hergestellt ist.

Figur 14 zeigt das Injektionsgerät im gekoppelten Zustand, d.h. im Kupplungseingriff.

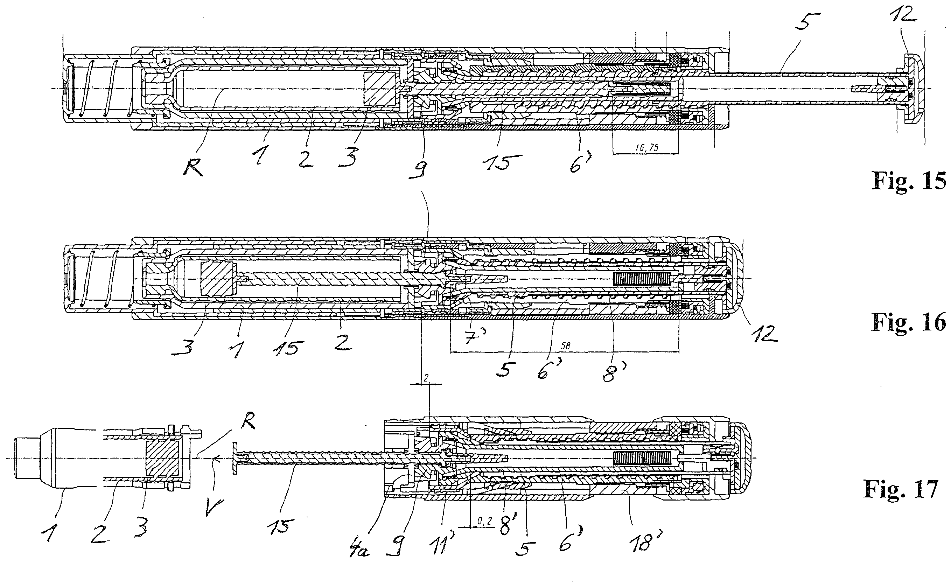

Die Figuren 15 und 16 entsprechen den Figuren 6 und 7 des ersten Ausführungsbeispiels, so dass darauf verwiesen werden kann.

Figur 17 zeigt das Injektionsgerät des zweiten Ausführungsbeispiels während des Austauschs des Reservoirs 2. Nachdem das Reservoir 2, wie in Figur 16 gezeigt, entleert ist, wird das Gehäuseteil 1 von dem Gehäuseteil 4 gelöst, wodurch das Entkopplungsglied 11' in die Entkopplungsposition bewegt wird. Diese Funktion entspricht vollkommen derjenigen des Entkopplungsglieds 11 des ersten Ausführungsbeispiels, so dass auf die dortigen Erläuterungen und die Figuren 8-10 verwiesen werden kann.

In dem in Figur 17 gezeigten Zustand nimmt das Gehäuseteil 1 bereits das neue Reservoir 2 auf. Um das Gehäuseteil 1 mit dem Gehäuseteil 4 zu verbinden, kann das Gehäuseteil 1 mit dem das Reservoir 2 proximal verschließenden Kolben 3 auf das Gehäuseteil 4 zu bewegt werden. Die frei aus dem Gehäuseteil 4 vorragende Kolbenstange 15 wird durch den andrückenden Kolben 3 im Gewindeeingriff mit dem frei drehbaren, aber axial festgelegten Kupplungsausgangsglied 9 zurückbewegt. Wegen der verdrehgesicherten Linearführung 4a, die im zweiten Ausführungsbeispiel von einer verdrehgesichert in das Gehäuseteil 4 eingesetzten Kupplungsaufnahme gebildet wird, vollführt die Kolbenstange 15 bei dem Zurückschieben eine axiale Linearbewegung, während das Kupplungsausgangsglied 9 gemeinsam mit der Kupplungshülse 8' um die gemeinsame Gewindeachse frei dreht. Anstatt die Kolbenstange 15 gegen den Kolben 3 drückend zurück zu bewegen, kann die Kolbenstange 15 auch vorher durch Druck unmittelbar auf ihren Stempel zurückbewegt werden.

Figur 18 zeigt den Kupplungsbereich mit dem in der Entkopplungsposition befindlichen Entkopplungsglied 11' im Detail. Die Funktion des Entkopplungsglieds 11' entspricht derjenigen des ersten Ausführungsbeispiels, nämlich Blockierung des Kupplungseingangsglieds 6' in der abgerückten Axialposition.

Im zweiten Ausführungsbeispiel werden die Dosierbewegung und die Antriebsbewegung ebenfalls über das Kupplungseingangsglied 6' und ein Anzeigekopplungsglied 22 in ein Getriebe der Dosisanzeige 20' eingeleitet. Auch das Anzeigekopplungsglied 22 ist verdrehgesichert mit dem Kupplungseingangsglied 6' verbunden und relativ zu dem Gehäuseteil 4 in und gegen die Richtung der Kupplungsbewegung X nicht beweglich.

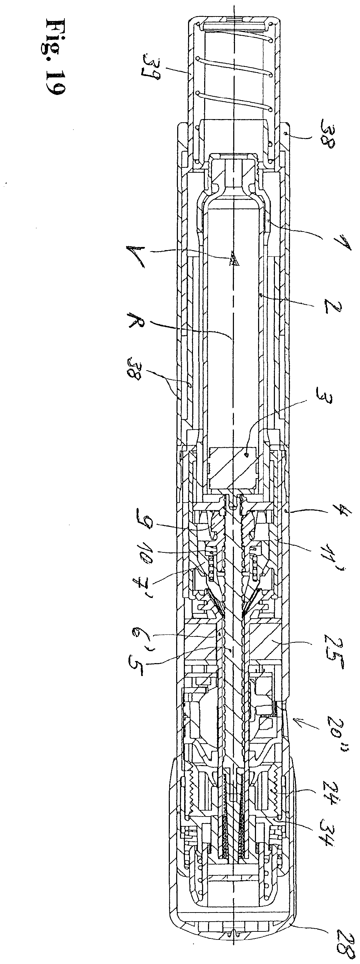

Die Figuren 19 bis 24 zeigen als drittes Ausführungsbeispiel ein Injektionsgerät, bei dem bei der Verabreichung die Antriebskraft für die Produktausschüttung nicht manuell, sondern von einem Antriebsglied 25 aufgebracht wird, das als Antriebsfeder gebildet ist. Das Antriebsglied 25 wird durch Einstellung der zu verabreichenden Dosis gespannt. Die bei der Dosiseinstellung aufgenommene Federenergie wird bei Auslösung des Geräts freigesetzt und in den Vortrieb der Kolbenstange 15 umgewandelt.

Figur 19 zeigt das Injektionsgerät des dritten Ausführungsbeispiels komplett mit dem montierten Gehäuseteil 38 und dem darin entgegen der Vortriebsrichtung V gegen die Kraft einer Rückstellfeder gleitverschiebbar aufgenommenen Nadelschutz 39.

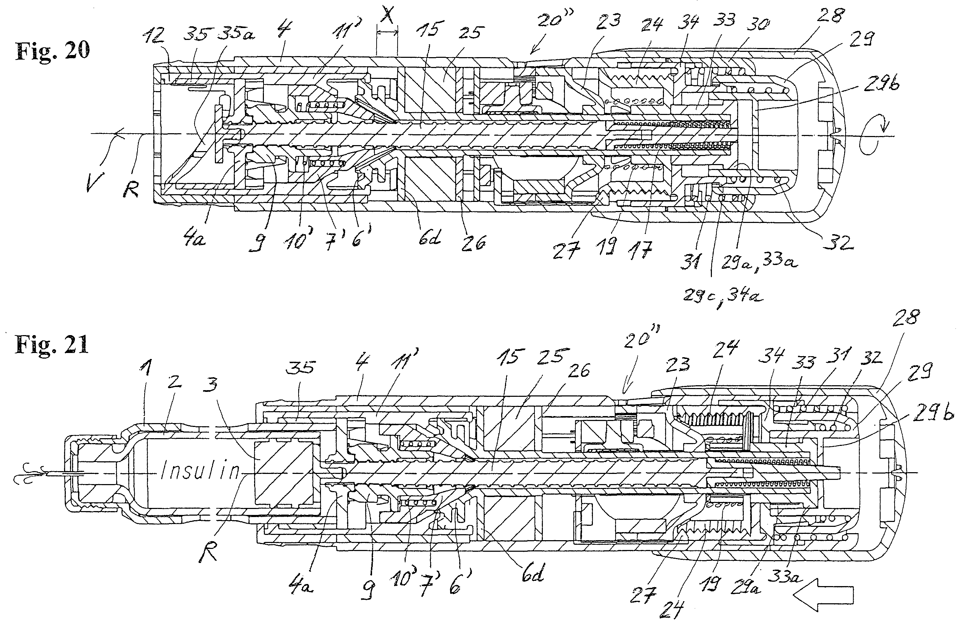

Die Figuren 20 und 21 zeigen das Gehäuseteil 4 mit den darin aufgenommenen Komponenten des Injektionsgeräts, Figur 20 in einem den vorhergehenden Ausführungsbeispielen vergleichbaren Ruhezustand, in dem die Dosis eingestellt werden kann, und Figur 21 im Kupplungseingriff. Soweit nachfolgend nichts anderes gesagt wird, werden insbesondere die Figuren 20 und 21 in Bezug genommen.

Das Antriebsglied 25 ist eine als Drehfeder wirkende Spiralfeder mit Federwindungen, die um die Gewindeachse R des Gewindeeingriffs zwischen dem Kupplungsausgangsglied 9 und der Kolbenstange 15 umlaufen. Die Federwindungen sind radial zu der Gewindeachse R übereinander angeordnet; sie weisen zu der Gewindeachse die Steigung Null auf. Ein inneres Ende der Federwindungen ist an dem Kupplungseingangsglied 6' befestigt, und ein äußeres Ende ist an einer Stützstruktur 26 befestigt, die mit dem Gehäuseteil 4 in Richtung der Kupplungsbewegung X bewegbar, aber verdrehgesichert verbunden ist. Die Stützstruktur 26 ist andererseits in und gegen die Richtung der Kupplungsbewegung X nicht bewegbar mit dem Kupplungseingangsglied 6' verbunden. Das Kupplungseingangsglied 6' ist relativ zu der Stützstruktur 26 um die Gewindeachse R drehbar. Eine weitere Stützstruktur 6d ist mit dem Kopplungseingangsglied 6' in und gegen die Richtung der Kupplungsbewegung X nicht bewegbar verbunden; im Ausführungsbeispiel sind das Kupplungseingangsglied 6' und die Stützstruktur 6d in einem Stück gebildet. Das Antriebsglied 25 wird von den Stützstrukturen 6d und 26 axial eingefasst.

Die Kupplung entspricht in ihrer Funktionsweise derjenigen des zweiten Ausführungsbeispiels, so dass für die Kupplungsglieder 6'-10' und das Entkopplungsglied 11' die gleichen Bezugszeichen verwendet werden. Im Unterschied zu der Kupplung des zweiten Ausführungsbeispiels ist allerdings die dortige Kupplungshülse 8' entfallen. Das Kupplungszwischenglied 7' ist unmittelbar mit dem Kupplungsausgangsglied 9 in einem die rotatorische Antriebsbewegung des Kupplungseingangsglieds 6' auf das Kupplungsausgangsglied 9 übertragenden Eingriff.

Mit 20" ist eine Dosisanzeige bezeichnet, die über ein Anzeigekopplungsglied 23 mit dem Kupplungseingangsglied 6' gekoppelt ist, und wie bereits die Anzeigekopplungsglieder 21 und 22 der anderen Ausführungsbeispiele verdrehgesichert mit dem Kupplungseingangsglied 6' verbunden ist. In und gegen die Richtung der Kupplungsbewegung X ist das Anzeigekopplungsglied 23 relativ zu dem Gehäuseteil 4 nicht bewegbar. Wie bereits im ersten und zweiten Ausführungsbeispiel besteht die verdrehgesicherte Verbindung des Anzeigekopplungsglieds 23 sowohl im ausgekuppelten als auch im eingekuppelten Zustand der Vorrichtung.

Um das Kupplungseingangsglied 6' für die Einstellung der Dosis und während der Aufbewahrung an der rotatorischen Antriebsbewegung zu hindern und das Antriebsglied 25 im gespannten Zustand zu halten, ist zwischen dem Kupplungsgehäuse 6' und dem Gehäuseteil 4 eine Verdrehsperre gebildet. Die Verdrehsperre besteht in der dargestellten Halteposition der Kupplungsglieder 6', 7' und 9 zwischen einem ersten Sperrglied 24 und einem zweiten Sperrglied 34. Das Sperrglied 24 ist verdrehgesichert mit dem Kupplungseingangsglied 6' verbunden. Das Sperrglied 34 ist verdrehgesichert mit dem Gehäuseteil 4 verbunden, aber relativ zu dem Gehäuseteil 4 und dem Kupplungseingangsglied 6' in und gegen die Richtung der Kupplungsbewegung X bewegbar. Die Sperrglieder 24 und 34 bilden mit ihren im Sperreingriff einander kontaktierenden Stirnflächen eine Ratsche, die eine das Antriebsglied 25 spannende Rotationsbewegung des Kupplungseingangsglieds 6' zuläßt und eine Rotationsbewegung in die Gegenrichtung verhindert.

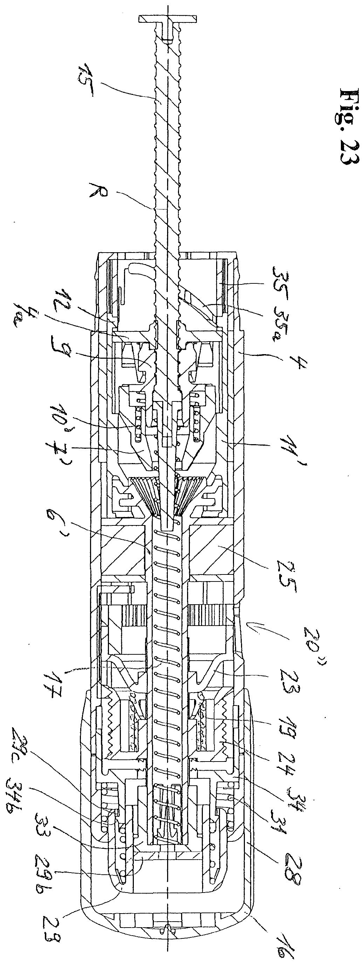

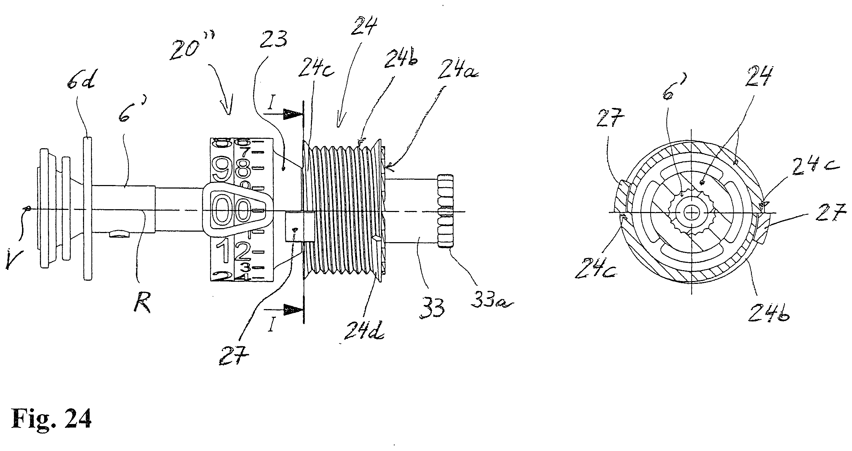

Figur 24 zeigt das Kupplungseingangsglied 6' mit dem darauf verdrehgesichert gelagerten Sperrglied 24, dem verdrehgesichert mit dem Kupplungseingangsglied 6' verbundenen Anzeigekopplungsglied 23 und einem mit dem Eingangsglied 6' nicht beweglich verbundenen Verbindungsteil 33. Das Anzeigekopplungsglied 23 bildet einen Einer-Zählring der Dosisanzeige 20" und ist mit einem Zehner-Zählring zur Anzeige der eingestellten Dosis in geeigneter Weise gekoppelt. Das Sperrglied 24 ist an einer dem Sperrglied 34 zugewandten, proximalen Stirnseite mit um die Achse R gleichmäßig angeordneten Sperrzähnen 24a versehen, die mit Gegenzähnen des Sperrglieds 34 im Sperreingriff zusammenwirken, um die Verdrehsperre bezüglich der Antriebsbewegung zu bilden. Das Sperrglied 24 ist für eine Zweitfunktion im Zusammenhang mit der Dosierung und Ausschüttung an einer Mantelaußenfläche mit einem Gewinde 24b versehen, dessen Gewindeachse mit der Gewindeachse R der Kolbenstange 15 zusammenfällt. In das Gewinde 24b greift ein Stoppglied 27 ein. Das Stoppglied 27 ist parallel zu der Gewindeachse R linear bewegbar geführt, im Ausführungsbeispiel in einer axialen Nut an der Innenmantelfläche des Gehäuseteils 4. Das Sperrglied 24 bildet für das Stoppglied 27 einen Verdrehanschlag 24c, der die den Vortrieb der Kolbenstange 15 bewirkende Antriebsbewegung des Kupplungseingangsglieds 6' begrenzt. Es bildet für das Stoppglied 27 einen weiteren Verdrehanschlag 24d, der die ausschüttbare und einstellbare Maximaldosis bestimmt. Auf der anderen Seite der Gewindeachse R ist dem in der Ansicht der Figur 23 erkennbaren Stoppglied 27 gegenüberliegend ein weiteres Stoppglied 27 angeordnet, das in gleicher Weise mit zwei weiteren Verdrehanschlägen 24c und 24d zusammenwirkt. Das Gewinde 24d ist doppelgängig. Die Stoppglieder 27 gelangen gleichzeitig auf Anschlag gegen die jeweils zugeordneten Verdrehanschläge 24c und 24d, wie in der Querschnittsdarstellung der Figur 23 für die Verdrehanschläge 24c erkennbar ist. Die Verdrehanschläge 24c bestimmen eine Nulldosisposition und die Verdrehanschläge 24d eine Maximaldosisposition.

Die Halteeinrichtung ist im dritten Ausführungsbeispiel in einer dritten Variante gebildet. Sie umfasst ein Rückstellglied 19, ferner das Anzeigekopplungsglied 23 und das Sperrglied 24. Das Rückstellglied 19 ist in Richtung der Kupplungsbewegung X über das Anzeigekopplungsglied 23 an dem Gehäuseteil 4 und gegen die Richtung der Kupplungsbewegung X an dem Sperrglied 24 abgestützt. Das Rückstellglied 19 drückt das Sperrglied 24 auf Anschlag gegen das Verbindungsteil 33. Da das Verbindungsteil 33 in und gegen die Richtung der Kupplungsbewegung X nicht beweglich mit dem Kupplungseingangsglied 6' verbunden ist, übt das Rückstellglied 19 somit über das Sperrglied 24 und das Verbindungsteil 33 auf das Kupplungseingangsglied 6' eine gegen die Richtung der Kupplungsbewegung X wirkende elastische Rückstellkraft aus, die das Kupplungseingangsglied 6' in der aus dem Kupplungseingriff ausgerückten Halteposition hält. Es wirkt wieder als Druckfeder. Das Sperrglied 24 ist ein Hülsenteil mit einem das Gewinde 24b bildenden äußeren Mantel, einem der verdrehgesicherten Lagerung auf dem Kupplungseingangsglied 6' dienenden inneren Mantel und einem die beiden Mäntel verbindenden Boden, an dem die Sperrzähne 24a gebildet sind. In das derart topfförmige Sperrglied 24 ragt das Rückstellglied 19 und stützt sich an dem Boden des Sperrglieds 24 ab.

Das Rückstellglied 19 drückt das Sperrglied 24 nicht nur auf Anschlag gegen das Verbindungsteil 33, sondern auch auf einen Anschlag gegen das Gehäuseteil 4. Dieser weitere Anschlag verhindert, dass das Sperrglied 24 über die in Figur 20 eingenommene Halteposition hinaus gegen die Richtung der Kupplungsbewegung X bewegt werden kann. Das Sperrglied 24 ist somit relativ zu dem Kupplungseingangsglied 6' gegen die rückstellende Elastizitätskraft des Rückstellglieds 19 in Richtung der Kupplungsbewegung X bewegbar. Umgekehrt ist das Kupplungseingangsglied 6' relativ zu dem auf Anschlag gegen das Gehäuseteil 4 liegenden Sperrglied 24 gegen die Richtung der Kupplungsbewegung X bewegbar.

Die zwischen der Kolbenstange 15 und dem Verbindungsteil 33 gespannte Ausgleichsfeder 17 unterstützt das Rückstellglied 19 bei dessen Funktion, das Kupplungseingangsglied 6' in der Halteposition zu halten. Die Ausgleichsfeder 17 könnte für das Ausrücken der Kupplungsglieder 6', 7' und 9 im Grunde das Rückstellglied 19 ersetzen. Bevorzugt ist sie jedoch so schwach, dass sie zumindest nach einer teilweisen Entspannung die Kupplungsglieder 6'-9 nicht mehr mit ausreichender Sicherheit in der Halteposition und die Kupplung somit im ausgekuppelten Zustand halten kann.

Für die Auslösung des Antriebsglieds 25 ist ein Auslöseelement 28 vorgesehen. Das Auslöseelement 28 ist relativ zu dem Gehäuseteil 4 in Richtung der Kupplungsbewegung X, im Ausführungsbeispiel die Vortriebsrichtung V bzw. die distale Richtung, translatorisch und um die Rotationsachse R des Kupplungseingangsglieds 6', die im Ausführungsbeispiel mit der Gewindeachse R der Kolbenstange 15 zusammenfällt, rotatorisch bewegbar und wird bei diesen beiden Bewegungen von dem Gehäuseteil 4 geführt. Durch die translatorische Bewegung in die distale Richtung werden der Kupplungseingriff zwischen dem Kupplungseingangsglied 6' und dem Kupplungszwischenglied 7' hergestellt und die Verdrehsperre zwischen den Sperrgliedern 24 und 34 gelöst und das Antriebsglied 25, d.h. die Ausschüttung, hierdurch ausgelöst. Die translatorische Bewegung in die Vortriebsrichtung V wird im Folgenden daher auch als Auslösebewegung bezeichnet.

Das Auslöseelement 28 bildet in einer weiteren Funktion das Dosierglied des dritten Ausführungsbeispiels. Durch die rotatorische Bewegung des Auslöseelements 28 relativ zu dem Gehäuseteil 4 wird über mehrere Zwischenglieder die mit dem nächsten Ausschüttvorgang ausschüttbare Produktdosis eingestellt. Diese Bewegung wird im Folgenden auch als Dosierbewegung bezeichnet. Aus der Nulldosisposition, die in Figur 20 dargestellt ist und durch Anschlag der Stoppglieder 27 an den die Antriebsbewegung des Kupplungseingangsglieds 6' begrenzenden Verdrehanschlägen 29c des Sperrglieds 24 bestimmt wird, kann die Dosis durch Verdrehen des Auslöseelements 28 in Richtung des eingezeichneten Drehrichtungspfeils, die Dosierrichtung, eingestellt werden. Die rotatorische Dosierbewegung des Auslöseelements 28 wird über ein Innenteil 29, das verdreh- und verschiebegesichert mit dem Auslöseelement 28 verbunden oder einstückig damit gebildet ist, und das Verbindungsteil 33 auf das Kupplungseingangsglied 6' übertragen. Für die Übertragung sind das Innenteil 29 und das Verbindungsteil 33 miteinander in einem verdrehgesicherten Eingriff, und das Verbindungsteil 33 ist mit dem Kupplungseingangsglied 6' verdrehgesichert verbunden. Für die Verdrehsicherung sind das Innenteil 29 und das Verbindungsteil 33 mit einer Innenverzahnung 29a und einer Außenverzahnung 33a versehen, die im Ruhezustand des Geräts ineinander greifen und axial gegeneinander verschiebbar sind.

Das Auslöseelement 28 ist bedienerfreundlich im proximalen Endbereich des Gehäuseteils 4 angeordnet. Sein äußeres Hülsenteil umgibt das Gehäuseteil 4. Ein Boden des Auslöseelements 28 bildet ein proximales Ende des Injektionsgeräts. Für die Einstellung der Dosis ist das Auslöseelement 28 wie ein Drehknopf bedienbar und weist hierfür an seiner äußeren Mantelfläche eine Riffelung auf. Für die Auslösung ist es wie ein Druckknopf bedienbar. Bei der Dosierbewegung verrastet das Auslöseelement 28 den Dosiseinheiten entsprechend in diskreten Drehwinkelpositionen mit dem Gehäuseteil 4.

Von dem Innenteil 29 ragt ein Anschlagelement 29b einer proximalen Stirnfläche des Verbindungsteils 33 zugewandt nach radial einwärts ab. Im Ruhezustand des Geräts verbleibt zwischen dem Verbindungsteil 33 und dem Anschlagelement 29b ein lichter Abstand, der gerade so groß ist, dass bei der Auslösebewegung des Auslöseelements 28 die Verdrehsicherung zwischen dem Innenteil 29 und dem Verbindungsteil 33 sich löst, bevor das Anschlagelement 29b die Relativbewegung des Auslöselements 28 relativ zu dem Verbindungsteil 33 durch Anschlagkontakt beendet.

Das zweite Sperrglied 34 wird mittels einer Sperrfeder 31 in den Sperreingriff mit dem Sperrglied 24 gespannt. Die Sperrfeder 31 stützt sich hierfür in Richtung der Kupplungsbewegung X an dem Sperrglied 34 und der Kupplungsbewegung X entgegen an einem Gehäuseteil 30 ab, das mit dem Gehäuseteil 4 fest verbunden ist. Eine zwischen dem Innenteil 29 und dem Sperrglied 34 angeordnete weitere Feder 32 spannt das Auslöseelement 28 relativ zu dem Sperrglied 34 in eine proximale Endposition. Das Sperrglied 34 wird von dem Gehäuseteil 4 verdrehgesichert axial geführt. Das Gehäuseteil 4 bildet für die Bewegbarkeit des Sperrglieds 34 einen distalen und einen proximalen Anschlag.

In dem in Figur 20 dargestellten Ruhezustand stellt der Benutzer die Dosis ein, indem er das Auslöselement 28 in die Dosierrichtung verdreht. Das Auslöseelement 28 nimmt bei dieser rotatorischen Dosierbewegung über die Verdrehsicherung 29a, 33a das Verbindungsteil 33 mit, das seinerseits das Kupplungseingangsglied 6' mitnimmt, das somit die gleiche rotatorische Dosierbewegung wie das Auslöseelement 28 vollführt. Durch die Rotation des Kupplungseingangsglieds 6' wird das Antriebsglied 25 gespannt. Das Stoppglied 27 wandert im Eingriff mit dem Gewinde 24b des Sperrglieds 24 von dem die Nulldosis bestimmenden Anschlag 24c des Gewindes 24b in Richtung des die Maximaldosis bestimmenden Anschlags 24d (Fig. 24).

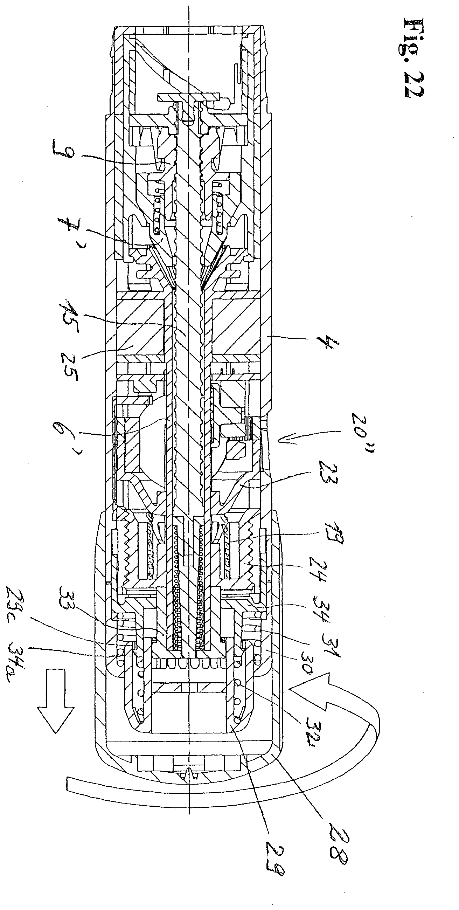

Das Injektionsgerät bietet auch eine bequeme Möglichkeit der Dosiskorrektur, wie anhand des Vergleichs der Figuren 20 und 22 deutlich wird. Sollte der Benutzer versehentlich eine zu hohe Dosis eingestellt haben, kann er die Dosis durch Zurückdrehen des Kupplungseingangsglieds 6' korrigieren. Für die Dosiskorrektur zieht er das Auslöseelement 28 in die proximale Richtung. Diese Ausrückbewegung des Auslöseelements 28 ist in Figur 22 durch einen Pfeil angedeutet, ebenso wie die Drehrichtung für die Korrektur. Das Innenteil 29 und das Sperrglied 34 sind in dem Ruhezustand des Geräts in Bezug auf eine Bewegung in die proximale Richtung in einem Mitnahmeeingriff. Die entsprechenden Mitnehmer sind mit 29c und 34a bezeichnet. Die von dem Innenteil 29 gebildeten Mitnehmer 29c und die von dem Sperrglied 34 gebildeten Mitnehmer 34a hintergreifen einander und bilden für die Ausrückbewegung des Auslöseelements 28 eine Verhakung. Durch Zug an dem Auslöseelement 28 wird somit das Sperrglied 34 gegen die Kraft der Sperrfeder 31 ebenfalls in die proximale Richtung bewegt und löst sich dabei aus dem Sperreingriff mit dem Sperrglied 24, das gegen das Gehäuseteil 4 auf Anschlag ist. Sobald die Verdrehsperre gelöst ist, kann der Benutzer mittels einer Rückdrehbewegung des Auslöseelements 28 und den nach wie vor bestehenden verdrehgesicherten Eingriff des Innenteils 29 mit dem Verbindungsteil 33 die Dosis korrigieren. Sobald der Benutzer das Auslöseelement 28 freigibt, schnappt es zusammen mit dem Sperrglied 34 unter der Wirkung der Sperrfeder 31 in die distale Richtung zurück und das Sperrglied 34 dadurch wieder in den Sperreingriff mit dem Sperrglied 24. Bei der Rückdrehbewegung hält der Benutzer zweckmäßigerweise weiterhin das Auslöseelement 28 fest, was durch die Drehwinkelrastpositionen des Auslöseelements 28 erleichtert wird. Grundsätzlich kann er es jedoch auch zurückschnappen lassen und gegebenenfalls wieder aufdosieren.

Nach Einstellung der gewünschten Dosis wird das Gerät an der gewünschten Stelle der Verabreichung auf die Haut aufgesetzt, und die Injektionsnadel wird eingestochen. Das Auslöseelement 28 übernimmt für das Einstechen der Nadel eine weitere Funktion, wofür es mit dem Nadelschutz 39 (Figur 19) gekoppelt ist.

In einer ersten Phase des Einstechens drückt der Benutzer das Injektionsgerät gegen die Haut, so dass sich der Nadelschutz 39 relativ zu dem Gehäuseteil 38 in die distale Richtung bewegt. Durch diesen ersten Teil der Bewegung des Nadelschutzes 39 wird die Injektionsnadel allerdings noch nicht freigelegt, ihre Spitze steht vielmehr weiterhin hinter dem Nadelschutz 39 zurück. Der Nadelschutz 39 gelangt in dieser ersten Phase des Einstechvorgangs auf Anschlag gegen ein Widerstandselement, so dass er sich relativ zu dem Gehäuseteil 38 nicht weiter in die distale Richtung bewegen kann. Bei weiterhin auf das Injektionsgerät in Richtung Haut ausgeübtem Druck drückt der Benutzer das Auslöseelement 28 in die proximale Richtung. Im Verlaufe dieser ersten Phase seiner Auslösebewegung löst das Auslöseelement 28 einen Anschlagkontakt zwischen dem Nadelschutz 39 und dem Widerstandselement, so dass das Injektionsgerät und damit zusammen die Injektionsnadel relativ zu dem Nadelschutz 39 in Richtung auf die Haut bewegt werden und die Injektionsnadel einsticht. Bezüglich der Funktion des Auslöseelements 28 für das Einstechen der Nadel wird die von der Anmelderin unter dem gleichen Anmeldedatum eingereichte Patentanmeldung "Aufsatzmodul für eine Injektionsvorrichtung mit einer Eingriffssteuerung für ein Nadelabdeckelement" in Bezug genommen.

Sobald die Injektionsnadel subkutan platziert ist, kann durch weiteren Druck auf das Auslöseelement 28 das Antriebsglied 25 ausgelöst und die Ausschüttung bewirkt werden. In der zweiten Phase der Auslösebewegung des Auslöseelements 28, die sich an die Einstechphase anschließt, wird das Auslöseelement 28 und damit das Innenteil 29 gegen den Druck der Feder 32 relativ zu dem Verbindungsteil 33 weiter in die distale Richtung gedrückt, so dass sich die Verdrehsicherung 29a, 33a löst. Das Auslöseelement 28 kann leer drehen. Sobald die Verdrehsicherung 29a, 33a gelöst ist, gelangt das Anschlagelement 29b in Anschlagkontakt mit dem Verbindungsteil 33. In der sich nun anschließenden dritten Phase der Auslösebewegung drückt das Auslöseelement 28 über das Anschlagelement 29b das Verbindungsteil 33 und damit das Kupplungseingangsglied 6' in die Richtung der Kupplungsbewegung X, im Ausführungsbeispiel in die Vortriebsrichtung V. Unter der Einwirkung der Federkraft der Sperrfeder 31 folgt das Sperrglied 34 dieser Bewegung, bis es auf Anschlag gegen das Gehäuseteil 4 gelangt. Bevor das Sperrglied 34 die Anschlagposition erreicht, gelangt das Kupplungseingangsglied 6' in den Kupplungseingriff mit dem Kupplungszwischenglied 7'. Das Kupplungseingangsglied 6' drückt das Kupplungszwischenglied 7' gegen die Kraft des Rückstellglieds 10' aus dem reibschlüssigen Sperreingriff mit dem Entkopplungsglied 11'. Nachdem der Sperreingriff zwischen den Konusflächen der beiden Glieder 7' und 11' gelöst und damit der Kupplungseingriff vollständig hergestellt ist, gelangt das Sperrglied 34 auf Anschlag mit dem Gehäuseteil 4. In der sich nun anschließenden letzten Phase der Auslösebewegung drückt das Auslöseelement 28 über das Verbindungsteil 33 das Sperrglied 24 aus dem Sperreingriff mit dem Sperrglied 34.
Sobald die von den Sperrgliedern 24 und 34 gebildete Verdrehsperre gelöst ist, setzt aufgrund der Antriebskraft des Antriebsglieds 25 die rotatorische Antriebsbewegung des Kupplungseingangsglieds 6' ein und wird über den Kupplungseingriff auf das Kupplungsausgangsglied 9 übertragen. Wegen ihrer verdrehgesicherten Führung in der Linearführung 4a bewegt sich die Kolbenstange 15 im Gewindeeingriff mit dem Kupplungsausgangsglied 9 in die Vortriebsrichtung V, und es wird Produkt ausgeschüttet. Diese Ausschüttbewegung wird durch Anschlag des Stoppglieds 27 an dem die Nulldosis bestimmenden Anschlag 24c des Sperrglieds 24 beendet.

Figur 21 zeigt das Injektionsgerät für den Fall der Einstellung einer Nulldosis oder einer geringen Primingdosis im eingekuppelten Zustand nach dem Lösen der Verdrehsperre 24, 34, d.h. nachdem das Auslöseelement 28 die Auslösebewegung vollständig ausgeführt hat. Die vorstehend beschriebene Auslösesequenz läuft bei vorteilhafterweise kontinuierlich auf das Auslöseelement 28 ausgeübtem Druck automatisch ab, vom Einstechen bis zur vollständigen Ausschüttung der eingestellten Dosis.

Figur 23 zeigt das Injektionsgerät nach Entleerung des Behältnisses 2. Das Gehäuseteil 1 ist bereits von dem Gehäuseteil 4 abgenommen worden. Die Kolbenstange 15 nimmt ihre distalste Position ein. Das Entkopplungsglied 11' blockiert das Kupplungseingangsglied 6' in der von dem Kupplungszwischenglied 7' abgerückten Position. Die Funktionsweise des Entkopplungsglieds 11' entspricht derjenigen in den anderen Ausführungsbeispielen. Im Unterschied zu den beiden ersten Ausführungsbeispielen sind das Gehäuseteil 1 und das Entkopplungsglied 11' jedoch nicht unmittelbar miteinander in einem Führungseingriff, sondern über eine Adapterstruktur 36. Die Adapterstruktur 36 ist eine Hülse die in dem Gehäuseteil 4 in denen Verbindungsabschnitt in und gegen die Richtung der Kupplungsbewegung X fixiert ist, sich aber um die zentrale Längsachse R des Gehäuseteils 4 drehen kann. Die Adapterstruktur 35 bildet eine Führungskurve 36a entweder als Ausnehmung an oder Durchbrechung in ihrer dem Entkopplungsglied 11' zugewandten Mantelfläche. Die Führungskurve 35a weist den Verlauf eines Gewindeabschnitts auf. Die über den Umfang gemessene Länge und die in Bezug auf die zentrale Längsachse des Gehäuseteils 4 gemessene Steigung der Führungskurve 35a sind so bemessen, dass bei einer viertel bis halben Umdrehung der Adapterstruktur 35 relativ zu dem Entkopplungsglied 11' dieses in die in Figur 21 dargestellte Entkopplungsposition bewegt wird. Für die Erzeugung der Axialbewegung greift das Entkopplungsglied 11' mit seinem Eingriffselement 12 in die Führungskurve 35a. Insoweit wird auf die Ausführungen zum ersten Ausführungsbeispiel verwiesen.

Die Adapterstruktur 35 bildet bei dem Verbinden der Gehäuseteile 1 und 4 eine Linearführung für das Gehäuseteil 1. Das Gehäuseteil 1 wird in die Adapterstruktur 35 eingeschoben, wobei vorzugsweise ein leichter Reibschluss und dementsprechend eine Gleitführung für das Gehäuseteil 1 besteht. Das Gehäuseteil 1 ist relativ zu der Adapterstruktur 35 um die zentrale Längsachse des Gehäuseteils 4 nicht verdrehbar. Der entsprechend verdrehgesicherte Eingriff wird gleich zu Beginn des Einschiebens des Gehäuseteils 1 in die Adapterstruktur 35 hergestellt. Nachdem das Gehäuseteil 1 bis auf Anschlag gegen das Gehäuseteil 4, nämlich dessen Kupplungsaufnahme bei 4a, eingeschoben worden ist, wird das Gehäuseteil 1 relativ zu dem Gehäuseteil 4 verdreht und nimmt bei dieser Drehbewegung die Adapterstruktur 36 mit, bis das Eingriffselement 12 des Entkopplungsglieds 11' an dem Ende der Führungskurve 36a auf Anschlag gelangt.

Die Drehbewegung des Gehäuseteils 1 ist vorzugsweise erst in dessen axialen Anschlagposition möglich, wofür eine bis in die Anschlagposition wirksame Verdrehsicherung auch zwischen den Gehäuseteilen 1 und 4 gebildet sein kann.

Die im Führungseingriff bewirkte Bewegung des Entkopplungsglieds 11' weist eine axiale Länge auf, die größer ist als die Länge X der vollständigen Kupplungsbewegung. Das Entkopplungsglied 11' drückt bei seiner Entkopplungsbewegung das Kupplungseingangsglied 6' über dessen im Ruhezustand eingenommene Halteposition hinaus und blockiert es in dieser Entkopplungsposition. Das Kupplungseingangsglied 6' nimmt bei dieser erzwungenen Entkopplungsbewegung über das Anschlagelement 29b das Auslöseelement 28 mit. Über die Verhakung der Mitnehmer 29c und 34a wird das Sperrglied 34 gegen die Kraft der Sperrfeder 31 ebenfalls mitgenommen und aus dem Sperreingriff bewegt. Das Sperrglied 24 kann dem Sperrglied 34 nicht folgen, da es gegen das Gehäuseteil 4 auf Anschlag ist. Durch das Lösen der Gehäuseteile 1 und 4 wird somit mittels des Entkopplungsmechanismus, den die Gehäuseteile 1 und 4 über die Adapterstruktur 35 mit dem Entkopplungsglied 11' bilden, die Verdrehsperre gelöst. Sollte das Kupplungseingangsglied 6' noch nicht die Nulldosisposition einnehmen, wird es von dem Antriebsglied 25 spätestens jetzt in die Nulldosisposition verdreht und die Dosisanzeige 20" genullt. In diesem Zusammenhang sei wiederholt auf den besonderen Vorteil der Kopplung zwischen der Dosisanzeige 20" und dem Kupplungseingangsglied 6' hingewiesen, dass nämlich bei jeder Ausschüttung die Dosisanzeige 20" der ausgeschütteten Dosis entsprechend zurückgesetzt wird. Sollte die eingestellte Dosis einmal nicht ausgeschüttet worden sein, weil beispielsweise der Injektionsvorgang abgebrochen wurde oder das Behältnis 2 nicht die eingestellte Dosis vollständig mehr enthielt, kann der Benutzer dies an der in diesem Fall nur teilweise zurückgesetzten Dosisanzeige 20" ablesen.

### Bezugszeichen:

- 1: erstes Gehäuseteil
- 1a: Führungskurve
- 2: Behältnis
- 3: Kolben
- 4: zweites Gehäuseteil
- 4a: Linearführung
- 4b: Linearfiihrung
- 4c: Umfangsführung
- 4d: Fixierelement
- 4e: Axialführung
- 5: Antriebsglied
- 6, 6': Kupplungseingangsglied
- 6a: Eingriffselement
- 6b: Eingriffselement
- 6c: Stützstruktur
- 6d: Stützstruktur
- 7, 7': Kupplungszwischenglied
- 7a: Eingriffselement
- 7b: Eingriffselement
- 7c: Eingriffselement
- 8, 8': Kupplungshülse
- 8a: Eingriffselement
- 9: Kupplungsausgangsglied
- 10: Kupplungs-Rückstellglied
- 11, 11': Entkopplungsglied
- 11a: Eingriffselement
- 12: Eingriffselement
- 13: Fixierelement
- 14: Kupplungs-Rückstellglied
- 15: Kolbenstange
- 15a: Teller
- 16: Auslöseelement
- 17: Ausgleichsfeder
- 18, 18': Dosierglied
- 18a: Eingriffselement
- 19: Kupplungs-Rückstellglied
- 20, 20' 20": Dosisanzeige
- 21: Anzeigekopplungsglied
- 22: Anzeigekopplungsglied
- 23: Anzeigekopplungsglied
- 24: erstes Sperrglied
- 24a: Sperrzähne
- 24b: Gewinde
- 24c: Verdrehanschlag
- 24d: Verdrehanschlag
- 25: Antriebsglied
- 26: Stützstruktur
- 27: Stoppglied
- 28: Auslöseelement
- 29: Innenteil
- 29a: Verzahnung
- 29b: Anschlagelement
- 29c: Mitnehmer
- 30: Gehäuseteil
- 31: Sperrfeder
- 32: Feder
- 33: Verbindungsteil
- 33a: Verzahnung
- 34: zweites Sperrglied
- 34a: Mitnehmer
- 35: Adapterstruktur
- 35a: Führungskurve
- 36: -
- 37: Schutzkappe
- 38: Gehäuseteil
- 39: Nadelschutz

## Patentansprüche

1. Vorrichtung zur Verabreichung eines fluiden Produkts, die Vorrichtung umfassend:
a) ein erstes Gehäuseteil (1) mit einem Reservoir für das Produkt,
b) ein zweites Gehäuseteil (4), das mit dem ersten Gehäuseteil (1) verbunden ist,
c) eine zur Ausübung einer Ausschüttbewegung von dem zweiten Gehäuseteil (4) bewegbar gehaltene Kolbenstange (15),
d) ein betätigbares oder auslösbares Antriebsglied (25) für die Kolbenstange (15),
e) eine Kupplung (6'-11 ') mit einem Kupplungseingangsglied (6; 6'), das in einem Kupplungseingriff das Antriebsglied (25) mit der Kolbenstange (15) koppelt und eine Antriebskraft des Antriebsglieds (5) auf die Kolbenstange (15) überträgt und die Ausschüttbewegung bewirkt, [Anspruch 1]
f) wobei das Antriebselement (25) eine als Drehfeder wirkende Spiralfeder ist, [Seite 8, Absatz 3, Zeile 2]
g) und wobei die Vorrichtung ein Injektionsgerät ist zur Ausschüttung des fluiden Produkts durch eine in einen Auslass des Reservoirs (2) ragende Injektionsnadel. [Seite 11, Absatz 3, Zeile 6]

2. Vorrichtung nach Anspruch 1, wobei ein radial äusseres Ende der Spiralfeder (25) verdrehgesichert mit dem zweiten Gehäuseteil (4) verbunden ist. [Seite 8, Absatz 3, Zeile 9]

3. Vorrichtung nach Anspruch 1, wobei die Spiralfeder (25) Windungen aufweist welche um eine Gewindeachse R der Kolbenstange (15) umlaufen. [Seite 25, Absatz 5, Zeile 1 bis 3]

4. Vorrichtung nach Anspruch 1, umfassend ein bei der Produktausschüttung von dem Antriebsglied (25) angetriebenes Kupplungsausgangsglied in Gewindeeingriff mit der Kolbenstange (15). [Seite 7, Absatz 1, Zeile 1, 7]

5. Vorrichtung nach Anspruch 4, wobei die Kolbenstange (15) auf ihrer axialen Länge mit einem Aussengewinde versehen ist. [Seite 12, Absatz 3, Zeile 1]

6. Vorrichtung nach einem der vorhergehenden Ansprüche, wobei die Injektionsnadel an dem distalen Ende des ersten Gehäuseteils (1) befestigt ist. [Seite 11, Absatz 3, Zeile 5]

7. Vorrichtung nach einem der vorhergehenden Ansprüche, **dadurch gekennzeichnet, dass** die Kolbenstange (15) verdrehgesichert linear geführt ist. [Anspruch 26]

8. Vorrichtung nach einem der vorhergehenden Ansprüche, wobei die Spiralfeder durch Einstellung einer zu verabreichenden Dosis gespannt wird. [Seite 31, Absatz 1, Zeile 6]

9. Vorrichtung nach einem der vorhergehenden Ansprüche, umfassend ein mit dem zweiten Gehäuseteil (4) bewegbar verbundenes Entkopplungsglied (11'), das mit dem ersten Gehäuseteil (1) so gekoppelt ist, dass es durch eine beim Lösen der Gehäuseteile (1, 4) stattfindende Bewegung der Gehäuseteile (1,4) relativ zueinander in eine Entkopplungsposition bewegt wird, in der es die Kolbenstange (15) von dem Kupplungseingangsglied (6') entkoppelt. [Anspruch 1]

10. Vorrichtung nach einem der vorhergehenden Ansprüche, ferner umfassend eine Dosisanzeige (20') zum Anzeigen einer einstellbaren Produktdosis, wobei die Dosisanzeige (20') mit dem Antriebsglied (25) so gekoppelt ist, dass eine Bewegung, die das Kupplungseingangsglied (6') bei einer Einstellung der Produktdosis ausführt, eine Änderung der angezeigten Produktdosis bewirkt, und wobei ein Entkopplungsglied (11') in der Entkopplungsposition die Dosisanzeige (20') von der Kolbenstange (15) entkoppelt. [Anspruch 2]
